# EUROPEAN PATENT SPECIFICATION

(11) **EP 3 370 777 B1**
(45) Date of publication and mention of the grant of the patent: **29.07.2020**
(21) Application number: 15807696.8
(22) Date of filing: 06.11.2015
(51) Int. Cl.: A61K 47/50, C07K 14/21

(54) **PURIFICATION METHOD FOR RECOMBINANT EXOTOXIN A, RECOMBINANT EXOTOXIN A MUTEINS AND RECOMBINANT PROTEINS COMPRISING EXOTOXIN A FRAGMENTS**
REINIGUNGSVERFAHREN FÜR REKOMBINANTES EXOTOXIN A, MUTEINE VON REKOMBINANTEM EXOTOXIN A UND REKOMBINANTE PROTEINE MIT FRAGMENTEN VON EXOTOXIN A
PROCÉDÉ DE PURIFICATION D'EXOTOXINE A RECOMBINANTE, MUTÉINES D'EXOTOXINE A RECOMBINANTE, ET PROTÉINES RECOMBINANTES COMPRENANT DES FRAGMENTS D'EXOTOXINE A

(43) Date of publication of application: 12.09.2018
(73) Proprietor: Uniwersytet Warszawski, 00-927 Warszawa (PL); Instytut Biologii Medycznej Polskiej Akademii Nauk, 93-232 Lodz (PL); Instytut Bioinfobank SP. Z O.O., 61-809 Poznan (PL)
(72) Inventor: BOROWIEC, Marta Zofia, 92-439 Lódz (PL); GRZESIK, Joanna, 23-204 Krasnik (PL); WALCZAK-DRZEWIECKA, Aurelia, 92-777 Lódz (PL); GORZKIEWICZ, Micha, 92-311 Lódz (PL); RODAKOWSKA, Ewelina, 62-025 Kostrzyn (PL); DASTYCH, Jaroslaw, 92-009 Lódz (PL); RYCHLEWSKI, Leszek, 60-744 Poznan (PL); GINALSKI, Krzysztof, 02-309 Warszawa (PL)
(74) Representative: Grzelak, Anna
(86) International application number: PCT/IB2015/058571
(87) International publication number: WO 2017/077367

(56) References cited:
- WO-A2-2006/001023
- US-A1- 2009 203 884
- WANG ET AL: "Expression, purification, and characterization of an immunotoxin containing a humanized anti-CD25 single-chain fragment variable antibody fused to a modified truncated Pseudomonas exotoxin A", PROTEIN EXPRESSION AND PURIFICATION, ACADEMIC PRESS, SAN DIEGO, CA, vol. 58, no. 1, 28 January 2008 (2008-01-28), pages 140-147, XP022435858, ISSN: 1046-5928, DOI: 10.1016/J.PEP.2007.09.009
- ANUPAM SINGH ET AL: "Protein recovery from inclusion bodies of Escherichia coli using mild solubilization process", MICROBIAL CELL FACTORIES, BIOMED CENTRAL, vol. 14, no. 1, 25 March 2015 (2015-03-25) , page 41, XP021220426, ISSN: 1475-2859, DOI: 10.1186/S12934-015-0222-8
- BRYAN K. BEATTIE ET AL: "In Vitro Enzyme Activation and Folded Stability of Pseudomonas aeruginosa Exotoxin A and Its C-Terminal Peptide +", BIOCHEMISTRY, vol. 35, no. 28, 1 January 1996 (1996-01-01), pages 9042-9051, XP055260033, US ISSN: 0006-2960, DOI: 10.1021/bi960396k

### SEQUENCE LISTING

<110> University of Warsaw
   Institute of Medical Biology PAS
   Institute BioInfoBank
<120> Method Of Purification Of Exotoxin A Proteins
<130> PZ/3355/AGR
<160> 16
<170> PatentIn version 3.5
<210> 1
   <211> 638
   <212> PRT
   <213> Pseudomonas aeruginosa
<220>
   <221> SIGNAL
   <222> (1)..(25)
<220>
   <221> DOMAIN
   <222> (26)..(277)
   <223> Domain IA
<220>
   <221> DOMAIN
   <222> (278)..(389)
   <223> Domain II
<220>
   <221> DOMAIN
   <222> (390)..(429)
   <223> Domain IB
<220>
   <221> DOMAIN
   <222> (430)..(638)
   <223> DOMAIN III
<400> 1
   Met His Leu Thr Pro His Trp Ile Pro Leu Val Ala Ser Leu Gly Leu
   1               5                   10                  15
   Leu Ala Gly Gly Ser Phe Ala Ser Ala Ala Glu Glu Ala Phe Asp Leu
               20                  25                  30
   Trp Asn Glu Cys Ala Lys Ala Cys Val Leu Asp Leu Lys Asp Gly Val
           35                  40                  45
   Arg Ser Ser Arg Met Ser Val Asp Pro Ala Ile Ala Asp Thr Asn Gly
       50                  55                  60
   Gln Gly Val Leu His Tyr Ser Met Val Leu Glu Gly Gly Asn Asp Ala
   65                  70                  75                  80
   Leu Lys Leu Ala Ile Asp Asn Ala Leu Ser Ile Thr Ser Asp Gly Leu
                   85                  90                  95
   Thr Ile Arg Leu Glu Gly Gly Val Glu Pro Asn Lys Pro Val Arg Tyr
               100                 105                 110
   Ser Tyr Thr Arg Gln Ala Arg Gly Ser Trp Ser Leu Asn Trp Leu Val
           115                 120                 125
   Pro Ile Gly His Glu Lys Pro Ser Asn Ile Lys Val Phe Ile His Glu
       130                 135                 140
   Leu Asn Ala Gly Asn Gln Leu Ser His Met Ser Pro Ile Tyr Thr Ile
   145                 150                 155                 160
   Glu Met Gly Asp Glu Leu Leu Ala Lys Leu Ala Arg Asp Ala Thr Phe
                   165                 170                 175
   Phe Val Arg Ala His Glu Ser Asn Glu Met Gln Pro Thr Leu Ala Ile
               180                 185                 190
   Ser His Ala Gly Val Ser Val Val Met Ala Gln Ala Gln Pro Arg Arg
           195                 200                 205
   Glu Lys Arg Trp Ser Glu Trp Ala Ser Gly Lys Val Leu Cys Leu Leu
       210                 215                 220
   Asp Pro Leu Asp Gly Val Tyr Asn Tyr Leu Ala Gln Gln Arg Cys Asn
   225                 230                 235                 240
   Leu Asp Asp Thr Trp Glu Gly Lys Ile Tyr Arg Val Leu Ala Gly Asn
                   245                 250                 255
   Pro Ala Lys His Asp Leu Asp Ile Lys Pro Thr Val Ile Ser His Arg
               260                 265                 270
   Leu His Phe Pro Glu Gly Gly Ser Leu Ala Ala Leu Thr Ala His Gln
           275                 280                 285
   Ala Cys His Leu Pro Leu Glu Thr Phe Thr Arg His Arg Gln Pro Arg
       290                 295                 300
   Gly Trp Glu Gln Leu Glu Gln Cys Gly Tyr Pro Val Gln Arg Leu Val
   305                 310                 315                 320
   Ala Leu Tyr Leu Ala Ala Arg Leu Ser Trp Asn Gln Val Asp Gln Val
                   325                 330                 335
   Ile Arg Asn Ala Leu Ala Ser Pro Gly Ser Gly Gly Asp Leu Gly Glu
               340                 345                 350
   Ala Ile Arg Glu Gln Pro Glu Gln Ala Arg Leu Ala Leu Thr Leu Ala
           355                 360                 365
   Ala Ala Glu Ser Glu Arg Phe Val Arg Gln Gly Thr Gly Asn Asp Glu
       370                 375                 380
   Ala Gly Ala Ala Ser Ala Asp Val Val Ser Leu Thr Cys Pro Val Ala
   385                 390                 395                 400
   Ala Gly Glu Cys Ala Gly Pro Ala Asp Ser Gly Asp Ala Leu Leu Glu
                   405                 410                 415
   Arg Asn Tyr Pro Thr Gly Ala Glu Phe Leu Gly Asp Gly Gly Asp Ile
               420                 425                 430
   Ser Phe Ser Thr Arg Gly Thr Gln Asn Trp Thr Val Glu Arg Leu Leu
           435                 440                 445
   Gln Ala His Arg Gln Leu Glu Glu Arg Gly Tyr Val Phe Val Gly Tyr
       450                 455                 460
   His Gly Thr Phe Leu Glu Ala Ala Gln Ser Ile Val Phe Gly Gly Val
   465                 470                 475                 480
   Arg Ala Arg Ser Gln Asp Leu Asp Ala Ile Trp Arg Gly Phe Tyr Ile
                   485                 490                 495
   Ala Gly Asp Pro Ala Leu Ala Tyr Gly Tyr Ala Gln Asp Gln Glu Pro
               500                 505                 510
   Asp Ala Arg Gly Arg Ile Arg Asn Gly Ala Leu Leu Arg Val Tyr Val
           515                 520                 525
   Pro Arg Ser Ser Leu Pro Gly Phe Tyr Arg Thr Gly Leu Thr Leu Ala
       530                 535                 540
   Ala Pro Glu Ala Ala Gly Glu Val Glu Arg Leu Ile Gly His Pro Leu
   545                 550                 555                 560
   Pro Leu Arg Leu Asp Ala Ile Thr Gly Pro Glu Glu Glu Gly Gly Arg
                   565                 570                 575
   Leu Glu Thr Ile Leu Gly Trp Pro Leu Ala Glu Arg Thr Val Val Ile
               580                 585                 590
   Pro Ser Ala Ile Pro Thr Asp Pro Arg Asn Val Gly Gly Asp Leu Asp
           595                 600                 605
   Pro Ser Ser Ile Pro Asp Lys Glu Gln Ala Ile Ser Ala Leu Pro Asp
       610                 615                 620
   Tyr Ala Ser Gln Pro Gly Lys Pro Pro Arg Glu Asp Leu Lys
   625                 630                 635
<210> 2
   <211> 613
   <212> PRT
   <213> Pseudomonas aeruginosa
<220>
   <221> DOMAIN
   <222> (1)..(252)
   <223> DOMAIN IA
<220>
   <221> DOMAIN
   <222> (253)..(364)
   <223> DOMAIN II
<220>
   <221> DOMAIN
   <222> (365)..(399)
   <223> DOMAIN IB
<220>
   <221> DOMAIN
   <222> (400)..(613)
   <223> DOMAIN III
<400> 2
   Ala Glu Glu Ala Phe Asp Leu Trp Asn Glu Cys Ala Lys Ala Cys Val
   1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
   85 90 95
   Trp Ser Leu Asn Trp Leu Val Pro Ile Gly His Glu Lys Pro Ser Asn
   100 105 110
   Ile Lys Val Phe Ile His Glu Leu Asn Ala Gly Asn Gln Leu Ser His
   115 120 125
   Met Ser Pro Ile Tyr Thr Ile Glu Met Gly Asp Glu Leu Leu Ala Lys
   130 135 140
   Leu Ala Arg Asp Ala Thr Phe Phe Val Arg Ala His Glu Ser Asn Glu
   145 150 155 160
   Met Gln Pro Thr Leu Ala Ile Ser His Ala Gly Val Ser Val Val Met
   165 170 175
   Ala Gln Ala Gln Pro Arg Arg Glu Lys Arg Trp Ser Glu Trp Ala Ser
   180 185 190
   Gly Lys Val Leu Cys Leu Leu Asp Pro Leu Asp Gly Val Tyr Asn Tyr
   195 200 205
   Leu Ala Gln Gln Arg Cys Asn Leu Asp Asp Thr Trp Glu Gly Lys Ile
   210 215 220
   Tyr Arg Val Leu Ala Gly Asn Pro Ala Lys His Asp Leu Asp Ile Lys
   225 230 235 240
   Pro Thr Val Ile Ser His Arg Leu His Phe Pro Glu Gly Gly Ser Leu
   245 250 255
   Ala Ala Leu Thr Ala His Gln Ala Cys His Leu Pro Leu Glu Thr Phe
   260 265 270
   Thr Arg His Arg Gln Pro Arg Gly Trp Glu Gln Leu Glu Gln Cys Gly
   275 280 285
   Tyr Pro Val Gln Arg Leu Val Ala Leu Tyr Leu Ala Ala Arg Leu Ser
   290 295 300
   Trp Asn Gln Val Asp Gln Val Ile Arg Asn Ala Leu Ala Ser Pro Gly
   305 310 315 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Ser Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Ile Ser Phe Ser Thr Arg Gly Thr Gln Asn
                   405                 410                 415
   Trp Thr Val Glu Arg Leu Leu Gln Ala His Arg Gln Leu Glu Glu Arg
               420                 425                 430
   Gly Tyr Val Phe Val Gly Tyr His Gly Thr Phe Leu Glu Ala Ala Gln
           435                 440                 445
   Ser Ile Val Phe Gly Gly Val Arg Ala Arg Ser Gln Asp Leu Asp Ala
       450                 455                 460
   Ile Trp Arg Gly Phe Tyr Ile Ala Gly Asp Pro Ala Leu Ala Tyr Gly
   465                 470                 475                 480
   Tyr Ala Gln Asp Gln Glu Pro Asp Ala Arg Gly Arg Ile Arg Asn Gly
                   485                 490                 495
   Ala Leu Leu Arg Val Tyr Val Pro Arg Ser Ser Leu Pro Gly Phe Tyr
               500                 505                 510
   Arg Thr Gly Leu Thr Leu Ala Ala Pro Glu Ala Ala Gly Glu Val Glu
           515                 520                 525
   Arg Leu Ile Gly His Pro Leu Pro Leu Arg Leu Asp Ala Ile Thr Gly
       530                 535                 540
   Pro Glu Glu Glu Gly Gly Arg Leu Glu Thr Ile Leu Gly Trp Pro Leu
   545                 550                 555                 560
   Ala Glu Arg Thr Val Val Ile Pro Ser Ala Ile Pro Thr Asp Pro Arg
                   565                 570                 575
   Asn Val Gly Gly Asp Leu Asp Pro Ser Ser Ile Pro Asp Lys Glu Gln
   580 585 590
   Ala Ile Ser Ala Leu Pro Asp Tyr Ala Ser Gln Pro Gly Lys Pro Pro
   595 600 605
   Arg Glu Asp Leu Lys
   610
<210> 3
   <211> 213
   <212> PRT
   <213> Pseudomonas aeruginosa
<220>
   <221> DOMAIN
   <222> (5)..(213)
   <223> DOMAIN III
<400> 3
   Leu Gly Asp Gly Gly Asp Ile Ser Phe Ser Thr Arg Gly Thr Gln Asn
   1               5                   10                  15
   Trp Thr Val Glu Arg Leu Leu Gln Ala His Arg Gln Leu Glu Glu Arg
               20                  25                  30
   Gly Tyr Val Phe Val Gly Tyr His Gly Thr Phe Leu Glu Ala Ala Gln
           35                  40                  45
   Ser Ile Val Phe Gly Gly Val Arg Ala Arg Ser Gln Asp Leu Asp Ala
       50                  55                  60
   Ile Trp Arg Gly Phe Tyr Ile Ala Gly Asp Pro Ala Leu Ala Tyr Gly
   65                  70                  75                  80
   Tyr Ala Gln Asp Gln Glu Pro Asp Ala Arg Gly Arg Ile Arg Asn Gly
                   85                  90                  95
   Ala Leu Leu Arg Val Tyr Val Pro Arg Ser Ser Leu Pro Gly Phe Tyr
               100                 105                 110
   Arg Thr Gly Leu Thr Leu Ala Ala Pro Glu Ala Ala Gly Glu Val Glu
           115                 120                 125
   Arg Leu Ile Gly His Pro Leu Pro Leu Arg Leu Asp Ala Ile Thr Gly
       130                 135                 140
   Pro Glu Glu Glu Gly Gly Arg Leu Glu Thr Ile Leu Gly Trp Pro Leu
   145                 150                 155                 160
   Ala Glu Arg Thr Val Val Ile Pro Ser Ala Ile Pro Thr Asp Pro Arg
                   165                 170                 175
   Asn Val Gly Gly Asp Leu Asp Pro Ser Ser Ile Pro Asp Lys Glu Gln
               180                 185                 190
   Ala Ile Ser Ala Leu Pro Asp Tyr Ala Ser Gln Pro Gly Lys Pro Pro
           195                 200                 205
   Arg Glu Asp Leu Lys
       210
<210> 4
   <211> 613
   <212> PRT
   <213> Artificial Sequence
<220>
   <223> Modified version of Pseudomonas aeruginosa Exotoxin A
<400> 4
   Ala Glu Glu Ala Phe Asp Leu Trp Asn Glu Cys Ala Lys Ala Cys Val
   1               5                   10                  15
   Leu Asp Leu Lys Asp Gly Val Arg Ser Ser Arg Met Ser Val Asp Pro
               20                  25                  30
   Ala Ile Ala Asp Thr Asn Gly Gln Gly Val Leu His Tyr Ser Met Val
           35                  40                  45
   Leu Glu Gly Gly Asn Asp Ala Leu Lys Leu Ala Ile Asp Asn Ala Leu
       50                  55                  60
   Ser Ile Thr Ser Asp Gly Leu Thr Ile Arg Leu Glu Gly Gly Val Glu
   65                  70                  75                  80
   Pro Asn Lys Pro Val Arg Tyr Ser Tyr Thr Arg Gln Ala Arg Gly Ser
                   85                  90                  95
   Trp Ser Leu Asn Trp Leu Val Pro Ile Gly His Glu Lys Pro Ser Asn
               100                 105                 110
   Ile Lys Val Phe Ile His Glu Leu Asn Ala Gly Asn Gln Leu Ser His
           115                 120                 125
   Met Ser Pro Ile Tyr Thr Ile Glu Met Gly Asp Glu Leu Leu Ala Lys
       130                 135                 140
   Leu Ala Arg Asp Ala Thr Phe Phe Val Arg Ala His Glu Ser Asn Glu
   145                 150                 155                 160
   Met Gln Pro Thr Leu Ala Ile Ser His Ala Gly Val Ser Val Val Met
                   165                 170                 175
   Ala Gln Ala Gln Pro Arg Arg Glu Lys Arg Trp Ser Glu Trp Ala Ser
               180                 185                 190
   Gly Lys Val Leu Cys Leu Leu Asp Pro Leu Asp Gly Val Tyr Asn Tyr
           195                 200                 205
   Leu Ala Gln Gln Arg Cys Asn Leu Asp Asp Thr Trp Glu Gly Lys Ile
       210                 215                 220
   Tyr Arg Val Leu Ala Gly Asn Pro Ala Lys His Asp Leu Asp Ile Lys
   225                 230                 235                 240
   Pro Thr Val Thr Ser His Arg Leu His Phe Pro Glu Gly Gly Ser Leu
                   245                 250                 255
   Ala Ala Leu Thr Ala His Gln Ala Cys His Leu Pro Leu Glu Thr Phe
               260                 265                 270
   Thr Arg His Arg Gln Pro Arg Gly Trp Glu Gln Leu Glu Gln Cys Gly
           275                 280                 285
   Tyr Pro Val Gln Arg Leu Val Ala Leu Tyr Leu Ala Ala Arg Leu Ser
       290                 295                 300
   Trp Asn Gln Val Asp Gln Val Ile Arg Asn Ala Leu Ala Ser Pro Gly
   305                 310                 315                 320
   Ser Gly Gly Asp Leu Gly Glu Ala Ile Arg Glu Gln Pro Glu Gln Ala
                   325                 330                 335
   Arg Leu Ala Leu Thr Leu Ala Ala Ala Glu Ser Glu Arg Phe Val Arg
               340                 345                 350
   Gln Gly Thr Gly Asn Asp Glu Ala Gly Ala Ala Ser Ala Asp Val Val
           355                 360                 365
   Ser Leu Thr Cys Pro Val Ala Ala Gly Glu Cys Ala Gly Pro Ala Asp
       370                 375                 380
   Ser Gly Asp Ala Leu Leu Glu Arg Asn Tyr Pro Thr Gly Ala Glu Phe
   385                 390                 395                 400
   Leu Gly Asp Gly Gly Asp Ile Ser Phe Ser Thr Arg Gly Thr Gln Asn
                   405                 410                 415
   Trp Thr Val Glu Arg Leu Leu Gln Ala His Arg Gln Leu Glu Glu Arg
               420                 425                 430
   Gly Tyr Val Phe Val Gly Tyr His Gly Thr Phe Leu Glu Ala Ala Gln
           435                 440                 445
   Ser Ile Val Phe Gly Gly Val Arg Ala Arg Ser Gln Asp Leu Asp Ala
       450                 455                 460
   Ile Trp Arg Gly Phe Tyr Ile Ala Gly Asp Pro Ala Leu Ala Tyr Gly
   465                 470                 475                 480
   Tyr Ala Gln Asp Gln Glu Pro Asp Ala Arg Gly Arg Ile Arg Asn Gly
                   485                 490                 495
   Ala Leu Leu Arg Val Tyr Val Pro Arg Ser Ser Leu Pro Gly Phe Tyr
               500                 505                 510
   Arg Thr Gly Leu Thr Leu Ala Ala Pro Glu Ala Ala Gly Glu Val Glu
           515                 520                 525
   Arg Leu Ile Gly His Pro Leu Pro Leu Arg Leu Asp Ala Ile Thr Gly
       530                 535                 540
   Pro Glu Glu Glu Gly Gly Arg Leu Glu Thr Ile Leu Gly Trp Pro Leu
   545                 550                 555                 560
   Ala Glu Arg Thr Val Val Ile Pro Ser Ala Ile Pro Thr Asp Pro Arg
                   565                 570                 575
   Asn Val Gly Gly Asp Leu Asp Pro Ser Ser Ile Pro Asp Lys Glu Gln
               580                 585                 590
   Ala Ile Ser Ala Leu Pro Asp Tyr Ala Ser Gln Pro Gly Lys Pro Pro
           595                 600                 605
   Arg Glu Asp Leu Lys
       610
<210> 5
   <211> 1845
   <212> DNA
   <213> Pseudomonas aeruginosa
<400> 5
   atggccgagg aagccttcga cctctggaac gaatgcgcca aggcctgcgt gctcgacctc 60
   aaggacggcg tgcgttccag ccgcatgagc gtcgacccgg ccatcgccga caccaacggc 120
   cagggcgtgc tgcactactc catggtcctg gagggcggca acgacgcgct caagctggcc 180
   atcgacaacg ccctcagcat caccagcgac ggcctgacca tccgcctcga aggtggcgtc 240
   gagccgaaca agccggtgcg ctacagctac acgcgccagg cgcgcggcag ttggtcgctg 300
   aactggctgg tgccgatcgg ccacgagaag ccttcgaaca tcaaggtgtt catccacgaa 360
   ctgaacgccg gtaaccagct cagccacatg tcgccgatct acaccatcga gatgggcgac 420
   gagttgctgg cgaagctggc gcgcgatgcc accttcttcg tcagggcgca cgagagcaac 480
   gagatgcagc cgacgctcgc catcagccat gccggggtca gcgtggtcat ggcccaggcc 540
   cagccgcgcc gggaaaagcg ctggagcgaa tgggccagcg gcaaggtgtt gtgcctgctc 600
   gacccgctgg acggggtcta caactacctc gcccagcagc gctgcaacct cgacgatacc 660
   tgggaaggca agatctaccg ggtgctcgcc ggcaacccgg cgaagcatga cctggacatc 720
   aagcccacgg tcactagtca tcgcctgcat ttccccgagg gcggcagcct ggccgcgctg 780
   accgcgcacc aggcctgcca cctgccgctg gagaccttca cccgtcatcg ccagccgcgc 840
   ggctgggaac aactggagca gtgcggctat ccggtgcagc ggctggtcgc cctctacctg 900
   gcggcgcggc tgtcgtggaa ccaggtcgac caggtgatcc gcaacgccct ggccagcccc 960
   ggcagcggcg gcgacctggg cgaagcgatc cgcgagcagc cggagcaggc ccgtctggcc 1020
   ctgaccctgg ccgccgccga gagcgagcgc ttcgtccggc agggcacagg caacgacgag 1080
   gccggcgcgg ccagcgccga cgtggtgagc ctgacctgcc cggtcgccgc cggtgaatgc 1140
   gcgggcccgg cggacagcgg cgacgccctg ctggagcgca actatcccac tggcgcggag 1200
   ttcctcggcg acggcggcga catcagcttc agcacccgcg gcacgcagaa ctggacggtg 1260
   gagcggctgc tccaggcgca ccgccaactg gaggagcgcg gctatgtgtt cgtcggctac 1320
   cacggcacct tcctcgaagc ggcgcaaagc atcgtcttcg gcggggtgcg cgcgcgcagc 1380
   caggacctcg acgcgatctg gcgcggtttc tatatcgccg gcgatccggc gctggcctac 1440
   ggctacgccc aggaccagga acccgacgcg cgcggccgga tccgcaacgg tgccctgctg 1500
   cgggtctatg tgccgcgctc gagtctgccg ggcttctacc gcaccggcct gaccctggcc 1560
   gcgccggagg cggcgggcga ggtcgaacgg ctgatcggcc atccgctgcc gctgcgcctg 1620
   gacgccatca ccggccccga ggaggaaggc gggcgcctgg agaccattct cggctggccg 1680
   ctggccgagc gcaccgtggt gattccctcg gcgatcccca ccgacccgcg caacgtcggc 1740
   ggcgacctcg acccgtccag catccccgac aaggaacagg cgatcagcgc cctgccggac 1800
   tacgccagcc agcccggcaa accgccgcgc gaggacctga agtga 1845
<210> 6
   <211> 7384
   <212> DNA
   <213> Artificial Sequence
<220>
   <223> pET28SUMO plasmid containing Exotoxin A sequence codon optimised for expression in E.coli.
<400> 6
   tggcgaatgg gacgcgccct gtagcggcgc attaagcgcg gcgggtgtgg tggttacgcg      60
   cagcgtgacc gctacacttg ccagcgccct agcgcccgct cctttcgctt tcttcccttc     120
   ctttctcgcc acgttcgccg gctttccccg tcaagctcta aatcgggggc tccctttagg     180
   gttccgattt agtgctttac ggcacctcga ccccaaaaaa cttgattagg gtgatggttc     240
   acgtagtggg ccatcgccct gatagacggt ttttcgccct ttgacgttgg agtccacgtt     300
   ctttaatagt ggactcttgt tccaaactgg aacaacactc aaccctatct cggtctattc     360
   ttttgattta taagggattt tgccgatttc ggcctattgg ttaaaaaatg agctgattta     420
   acaaaaattt aacgcgaatt ttaacaaaat attaacgttt acaatttcag gtggcacttt     480
   tcggggaaat gtgcgcggaa cccctatttg tttatttttc taaatacatt caaatatgta     540
   tccgctcatg aattaattct tagaaaaact catcgagcat caaatgaaac tgcaatttat     600
   tcatatcagg attatcaata ccatattttt gaaaaagccg tttctgtaat gaaggagaaa     660
   actcaccgag gcagttccat aggatggcaa gatcctggta tcggtctgcg attccgactc     720
   gtccaacatc aatacaacct attaatttcc cctcgtcaaa aataaggtta tcaagtgaga     780
   aatcaccatg agtgacgact gaatccggtg agaatggcaa aagtttatgc atttctttcc     840
   agacttgttc aacaggccag ccattacgct cgtcatcaaa atcactcgca tcaaccaaac     900
   cgttattcat tcgtgattgc gcctgagcga gacgaaatac gcgatcgctg ttaaaaggac     960
   aattacaaac aggaatcgaa tgcaaccggc gcaggaacac tgccagcgca tcaacaatat    1020
   tttcacctga atcaggatat tcttctaata cctggaatgc tgttttcccg gggatcgcag    1080
   tggtgagtaa ccatgcatca tcaggagtac ggataaaatg cttgatggtc ggaagaggca    1140
   taaattccgt cagccagttt agtctgacca tctcatctgt aacatcattg gcaacgctac    1200
   ctttgccatg tttcagaaac aactctggcg catcgggctt cccatacaat cgatagattg    1260
   tcgcacctga ttgcccgaca ttatcgcgag cccatttata cccatataaa tcagcatcca    1320
   tgttggaatt taatcgcggc ctagagcaag acgtttcccg ttgaatatgg ctcataacac    1380
   cccttgtatt actgtttatg taagcagaca gttttattgt tcatgaccaa aatcccttaa    1440
   cgtgagtttt cgttccactg agcgtcagac cccgtagaaa agatcaaagg atcttcttga    1500
   gatccttttt ttctgcgcgt aatctgctgc ttgcaaacaa aaaaaccacc gctaccagcg    1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacc atgggcagca gccatcatca tcatcatcac 5100
   agcagcggcc tggtgccgcg cggcagccat atggaaggtg aatatattaa actcaaagtc 5160
   attggacagg atagcagtga gattcacttc aaagtgaaaa tgacaacaca tctcaagaaa 5220
   ctcaaagaat catactgtca aagacagggt gttccaatga attcactcag gtttctcttt 5280
   gagggtcaga gaattgctga taatcatact ccaaaagaac tgggaatgga ggaagaagat 5340
   gtgattgaag tctaccagga acaaaccggt ggatccatgg ccgaggaagc cttcgacctc 5400
   tggaacgaat gcgccaaggc ctgcgtgctc gacctcaagg acggcgtgcg ttccagccgc 5460
   atgagcgtcg acccggccat cgccgacacc aacggccagg gcgtgctgca ctactccatg 5520
   gtcctggagg gcggcaacga cgcgctcaag ctggccatcg acaacgccct cagcatcacc 5580
   agcgacggcc tgaccatccg cctcgaaggt ggcgtcgagc cgaacaagcc ggtgcgctac 5640
   agctacacgc gccaggcgcg cggcagttgg tcgctgaact ggctggtgcc gatcggccac 5700
   gagaagcctt cgaacatcaa ggtgttcatc cacgaactga acgccggtaa ccagctcagc 5760
   cacatgtcgc cgatctacac catcgagatg ggcgacgagt tgctggcgaa gctggcgcgc 5820
   gatgccacct tcttcgtcag ggcgcacgag agcaacgaga tgcagccgac gctcgccatc 5880
   agccatgccg gggtcagcgt ggtcatggcc caggcccagc cgcgccggga aaagcgctgg 5940
   agcgaatggg ccagcggcaa ggtgttgtgc ctgctcgacc cgctggacgg ggtctacaac 6000
   tacctcgccc agcagcgctg caacctcgac gatacctggg aaggcaagat ctaccgggtg 6060
   ctcgccggca acccggcgaa gcatgacctg gacatcaagc ccacggtcac tagtcatcgc 6120
   ctgcatttcc ccgagggcgg cagcctggcc gcgctgaccg cgcaccaggc ctgccacctg 6180
   ccgctggaga ccttcacccg tcatcgccag ccgcgcggct gggaacaact ggagcagtgc 6240
   ggctatccgg tgcagcggct ggtcgccctc tacctggcgg cgcggctgtc gtggaaccag 6300
   gtcgaccagg tgatccgcaa cgccctggcc agccccggca gcggcggcga cctgggcgaa 6360
   gcgatccgcg agcagccgga gcaggcccgt ctggccctga ccctggccgc cgccgagagc 6420
   gagcgcttcg tccggcaggg cacaggcaac gacgaggccg gcgcggccag cgccgacgtg 6480
   gtgagcctga cctgcccggt cgccgccggt gaatgcgcgg gcccggcgga cagcggcgac 6540
   gccctgctgg agcgcaacta tcccactggc gcggagttcc tcggcgacgg cggcgacatc 6600
   agcttcagca cccgcggcac gcagaactgg acggtggagc ggctgctcca ggcgcaccgc 6660
   caactggagg agcgcggcta tgtgttcgtc ggctaccacg gcaccttcct cgaagcggcg 6720
   caaagcatcg tcttcggcgg ggtgcgcgcg cgcagccagg acctcgacgc gatctggcgc 6780
   ggtttctata tcgccggcga tccggcgctg gcctacggct acgcccagga ccaggaaccc 6840
   gacgcgcgcg gccggatccg caacggtgcc ctgctgcggg tctatgtgcc gcgctcgagt 6900
   ctgccgggct tctaccgcac cggcctgacc ctggccgcgc cggaggcggc gggcgaggtc 6960
   gaacggctga tcggccatcc gctgccgctg cgcctggacg ccatcaccgg ccccgaggag 7020
   gaaggcgggc gcctggagac cattctcggc tggccgctgg ccgagcgcac cgtggtgatt 7080
   ccctcggcga tccccaccga cccgcgcaac gtcggcggcg acctcgaccc gtccagcatc 7140
   cccgacaagg aacaggcgat cagcgccctg ccggactacg ccagccagcc cggcaaaccg 7200
   ccgcgcgagg acctgaagtg actcgagcac caccaccacc accactgaga tccggctgct 7260
   aacaaagccc gaaaggaagc tgagttggct gctgccaccg ctgagcaata actagcataa     7320
   ccccttgggg cctctaaacg ggtcttgagg ggttttttgc tgaaaggagg aactatatcc     7380
   ggat                                                                  7384
<210> 7
   <211> 626
   <212> PRT
   <213> Artificial Sequence
<220>
   <223> PEantiC immunotoxin in which an anti-CEA scFv is linked via a linker to a fragment of Exotoxin A (Domains II, IB and III).
<400> 7
   Gln Val Lys Leu Glu Gln Ser Gly Ala Gly Val Val Lys Pro Gly Ala
   1               5                   10                  15
   Ser Val Lys Leu Ser Cys Lys Ala Ser Gly Phe Asn Ile Lys Asp Ser
               20                  25                  30
   Tyr Met His Trp Leu Arg Gln Gly Pro Gly Gln Arg Leu Glu Trp Ile
           35                  40                  45
   Gly Trp Ile Asp Pro Glu Asn Gly Asp Thr Glu Tyr Ala Pro Lys Phe
       50                  55                  60
   Gln Gly Lys Ala Thr Phe Thr Thr Asp Thr Ser Ala Asn Thr Ala Tyr
   65                  70                  75                  80
   Leu Gly Leu Ser Ser Leu Arg Pro Glu Asp Thr Ala Val Tyr Tyr Cys
                   85                  90                  95
   Asn Glu Gly Thr Pro Thr Gly Pro Tyr Tyr Phe Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Glu Asn Val Leu Thr Gln Ser Pro Ser
       130                 135                 140
   Ser Met Ser Val Ser Val Gly Asp Arg Val Thr Ile Ala Cys Ser Ala
   145                 150                 155                 160
   Ser Ser Ser Val Pro Tyr Met His Trp Leu Gln Gln Lys Pro Gly Lys
                   165                 170                 175
   Ser Pro Lys Leu Leu Ile Tyr Leu Thr Ser Asn Leu Ala Ser Gly Val
               180                 185                 190
   Pro Ser Arg Phe Ser Gly Ser Gly Ser Gly Thr Asp Tyr Ser Leu Thr
           195                 200                 205
   Ile Ser Ser Val Gln Pro Glu Asp Ala Ala Thr Tyr Tyr Cys Gln Gln
       210                 215                 220
   Arg Ser Ser Tyr Pro Leu Thr Phe Gly Gly Gly Thr Lys Leu Glu Ile
   225                 230                 235                 240
   Lys Ala Ala Ala Gly Ser Ala Ser Gly Gly Gly Gly Ser Gly Gly Gly
                   245                 250                 255
   Thr Ser Gly Gly Gly Gly Ser Ala Glu Gly Gly Ser Leu Ala Ala Leu
               260                 265                 270
   Thr Ala His Gln Ala Cys His Leu Pro Leu Glu Thr Phe Thr Arg His
           275                 280                 285
   Arg Gln Pro Arg Gly Trp Glu Gln Leu Glu Gln Cys Gly Tyr Pro Val
       290                 295                 300
   Gln Arg Leu Val Ala Leu Tyr Leu Ala Ala Arg Leu Ser Trp Asn Gln
   305                 310                 315                 320
   Val Asp Gln Val Ile Arg Asn Ala Leu Ala Ser Pro Gly Ser Gly Gly
                   325                 330                 335
   Asp Leu Gly Glu Ala Ile Arg Glu Gln Pro Glu Gln Ala Arg Leu Ala
               340                 345                 350
   Leu Thr Leu Ala Ala Ala Glu Ser Glu Arg Phe Val Arg Gln Gly Thr
           355                 360                 365
   Gly Asn Asp Glu Ala Gly Ala Ala Ser Ala Asp Val Val Ser Leu Thr
       370                 375                 380
   Cys Pro Val Ala Ala Gly Glu Cys Ala Gly Pro Ala Asp Ser Gly Asp
   385                 390                 395                 400
   Ala Leu Leu Glu Arg Asn Tyr Pro Thr Gly Ala Glu Phe Leu Gly Asp
                   405                 410                 415
   Gly Gly Asp Ile Ser Phe Ser Thr Arg Gly Thr Gln Asn Trp Thr Val
               420                 425                 430
   Glu Arg Leu Leu Gln Ala His Arg Gln Leu Glu Glu Arg Gly Tyr Val
           435                 440                 445
   Phe Val Gly Tyr His Gly Thr Phe Leu Glu Ala Ala Gln Ser Ile Val
       450                 455                 460
   Phe Gly Gly Val Arg Ala Arg Ser Gln Asp Leu Asp Ala Ile Trp Arg
   465                 470                 475                 480
   Gly Phe Tyr Ile Ala Gly Asp Pro Ala Leu Ala Tyr Gly Tyr Ala Gln
                   485                 490                 495
   Asp Gln Glu Pro Asp Ala Arg Gly Arg Ile Arg Asn Gly Ala Leu Leu
               500                 505                 510
   Arg Val Tyr Val Pro Arg Ser Ser Leu Pro Gly Phe Tyr Arg Thr Gly
           515                 520                 525
   Leu Thr Leu Ala Ala Pro Glu Ala Ala Gly Glu Val Glu Arg Leu Ile
       530                 535                 540
   Gly His Pro Leu Pro Leu Arg Leu Asp Ala Ile Thr Gly Pro Glu Glu
   545                 550                 555                 560
   Glu Gly Gly Arg Leu Glu Thr Ile Leu Gly Trp Pro Leu Ala Glu Arg
                   565                 570                 575
   Thr Val Val Ile Pro Ser Ala Ile Pro Thr Asp Pro Arg Asn Val Gly
               580                 585                 590
   Gly Asp Leu Asp Pro Ser Ser Ile Pro Asp Lys Glu Gln Ala Ile Ser
           595                 600                 605
   Ala Leu Pro Asp Tyr Ala Ser Gln Pro Gly Lys Pro Pro Arg Glu Asp
       610                 615                 620
   Leu Lys
   625
<210> 8
   <211> 246
   <212> PRT
   <213> Artificial Sequence
<220>
   <223> Anti-CEA scFv as encoded by the PEantiC construct PEantiCP28S.
<400> 8
   Gln Val Lys Leu Glu Gln Ser Gly Ala Gly Val Val Lys Pro Gly Ala
   1               5                   10                  15
   Ser Val Lys Leu Ser Cys Lys Ala Ser Gly Phe Asn Ile Lys Asp Ser
               20                  25                  30
   Tyr Met His Trp Leu Arg Gln Gly Pro Gly Gln Arg Leu Glu Trp Ile
           35                  40                  45
   Gly Trp Ile Asp Pro Glu Asn Gly Asp Thr Glu Tyr Ala Pro Lys Phe
       50                  55                  60
   Gln Gly Lys Ala Thr Phe Thr Thr Asp Thr Ser Ala Asn Thr Ala Tyr
   65                  70                  75                  80
   Leu Gly Leu Ser Ser Leu Arg Pro Glu Asp Thr Ala Val Tyr Tyr Cys
                   85                  90                  95
   Asn Glu Gly Thr Pro Thr Gly Pro Tyr Tyr Phe Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Glu Asn Val Leu Thr Gln Ser Pro Ser
       130                 135                 140
   Ser Met Ser Val Ser Val Gly Asp Arg Val Thr Ile Ala Cys Ser Ala
   145                 150                 155                 160
   Ser Ser Ser Val Pro Tyr Met His Trp Leu Gln Gln Lys Pro Gly Lys
                   165                 170                 175
   Ser Pro Lys Leu Leu Ile Tyr Leu Thr Ser Asn Leu Ala Ser Gly Val
               180                 185                 190
   Pro Ser Arg Phe Ser Gly Ser Gly Ser Gly Thr Asp Tyr Ser Leu Thr
           195                 200                 205
   Ile Ser Ser Val Gln Pro Glu Asp Ala Ala Thr Tyr Tyr Cys Gln Gln
       210                 215                 220
   Arg Ser Ser Tyr Pro Leu Thr Phe Gly Gly Gly Thr Lys Leu Glu Ile
   225                 230                 235                 240
   Lys Ala Ala Ala Gly Ser
                   245
<210> 9
   <211> 18
   <212> PRT
   <213> Artificial Sequence
<220>
   <223> Linker between the anti-CEA scFv and fragment of Exotoxin A (Domains II, IB and III) as encoded by the PEantiC construct PEantiCP28S.
<400> 9
   Ala Ser Gly Gly Gly Gly Ser Gly Gly Gly Thr Ser Gly Gly Gly Gly
   1               5                   10                  15
   Ser Ala
<210> 10
   <211> 362
   <212> PRT
   <213> Artificial Sequence
<220>
   <223> Fragment of Exotoxin A (Domains II, IB and III) as encoded by the PEantiC construct PEantiCP28S
<400> 10
   Glu Gly Gly Ser Leu Ala Ala Leu Thr Ala His Gln Ala Cys His Leu
   1               5                   10                  15
   Pro Leu Glu Thr Phe Thr Arg His Arg Gln Pro Arg Gly Trp Glu Gln
               20                  25                  30
   Leu Glu Gln Cys Gly Tyr Pro Val Gln Arg Leu Val Ala Leu Tyr Leu
           35                  40                  45
   Ala Ala Arg Leu Ser Trp Asn Gln Val Asp Gln Val Ile Arg Asn Ala
       50                  55                  60
   Leu Ala Ser Pro Gly Ser Gly Gly Asp Leu Gly Glu Ala Ile Arg Glu
   65                  70                  75                  80
   Gln Pro Glu Gln Ala Arg Leu Ala Leu Thr Leu Ala Ala Ala Glu Ser
                   85                  90                  95
   Glu Arg Phe Val Arg Gln Gly Thr Gly Asn Asp Glu Ala Gly Ala Ala
               100                 105                 110
   Ser Ala Asp Val Val Ser Leu Thr Cys Pro Val Ala Ala Gly Glu Cys
           115                 120                 125
   Ala Gly Pro Ala Asp Ser Gly Asp Ala Leu Leu Glu Arg Asn Tyr Pro
       130                 135                 140
   Thr Gly Ala Glu Phe Leu Gly Asp Gly Gly Asp Ile Ser Phe Ser Thr
   145                 150                 155                 160
   Arg Gly Thr Gln Asn Trp Thr Val Glu Arg Leu Leu Gln Ala His Arg
                   165                 170                 175
   Gln Leu Glu Glu Arg Gly Tyr Val Phe Val Gly Tyr His Gly Thr Phe
               180                 185                 190
   Leu Glu Ala Ala Gln Ser Ile Val Phe Gly Gly Val Arg Ala Arg Ser
           195                 200                 205
   Gln Asp Leu Asp Ala Ile Trp Arg Gly Phe Tyr Ile Ala Gly Asp Pro
       210                 215                 220
   Ala Leu Ala Tyr Gly Tyr Ala Gln Asp Gln Glu Pro Asp Ala Arg Gly
   225                 230                 235                 240
   Arg Ile Arg Asn Gly Ala Leu Leu Arg Val Tyr Val Pro Arg Ser Ser
                   245                 250                 255
   Leu Pro Gly Phe Tyr Arg Thr Gly Leu Thr Leu Ala Ala Pro Glu Ala
               260                 265                 270
   Ala Gly Glu Val Glu Arg Leu Ile Gly His Pro Leu Pro Leu Arg Leu
           275                 280                 285
   Asp Ala Ile Thr Gly Pro Glu Glu Glu Gly Gly Arg Leu Glu Thr Ile
       290                 295                 300
   Leu Gly Trp Pro Leu Ala Glu Arg Thr Val Val Ile Pro Ser Ala Ile
   305                 310                 315                 320
   Pro Thr Asp Pro Arg Asn Val Gly Gly Asp Leu Asp Pro Ser Ser Ile
                   325                 330                 335
   Pro Asp Lys Glu Gln Ala Ile Ser Ala Leu Pro Asp Tyr Ala Ser Gln
               340                 345                 350
   Pro Gly Lys Pro Pro Arg Glu Asp Leu Lys
           355                 360
<210> 11
   <211> 7423
   <212> DNA
   <213> Artificial Sequence
<220>
   <223> DNA sequence of the PEantiC construct (PEantiCP28S)
<400> 11
   tggcgaatgg gacgcgccct gtagcggcgc attaagcgcg gcgggtgtgg tggttacgcg 60
   cagcgtgacc gctacacttg ccagcgccct agcgcccgct cctttcgctt tcttcccttc 120
   ctttctcgcc acgttcgccg gctttccccg tcaagctcta aatcgggggc tccctttagg 180
   gttccgattt agtgctttac ggcacctcga ccccaaaaaa cttgattagg gtgatggttc 240
   acgtagtggg ccatcgccct gatagacggt ttttcgccct ttgacgttgg agtccacgtt 300
   ctttaatagt ggactcttgt tccaaactgg aacaacactc aaccctatct cggtctattc 360
   ttttgattta taagggattt tgccgatttc ggcctattgg ttaaaaaatg agctgattta 420
   acaaaaattt aacgcgaatt ttaacaaaat attaacgttt acaatttcag gtggcacttt 480
   tcggggaaat gtgcgcggaa cccctatttg tttatttttc taaatacatt caaatatgta 540
   tccgctcatg aattaattct tagaaaaact catcgagcat caaatgaaac tgcaatttat 600
   tcatatcagg attatcaata ccatattttt gaaaaagccg tttctgtaat gaaggagaaa 660
   actcaccgag gcagttccat aggatggcaa gatcctggta tcggtctgcg attccgactc 720
   gtccaacatc aatacaacct attaatttcc cctcgtcaaa aataaggtta tcaagtgaga 780
   aatcaccatg agtgacgact gaatccggtg agaatggcaa aagtttatgc atttctttcc 840
   agacttgttc aacaggccag ccattacgct cgtcatcaaa atcactcgca tcaaccaaac 900
   cgttattcat tcgtgattgc gcctgagcga gacgaaatac gcgatcgctg ttaaaaggac 960
   aattacaaac aggaatcgaa tgcaaccggc gcaggaacac tgccagcgca tcaacaatat 1020
   tttcacctga atcaggatat tcttctaata cctggaatgc tgttttcccg gggatcgcag 1080
   tggtgagtaa ccatgcatca tcaggagtac ggataaaatg cttgatggtc ggaagaggca 1140
   taaattccgt cagccagttt agtctgacca tctcatctgt aacatcattg gcaacgctac 1200
   ctttgccatg tttcagaaac aactctggcg catcgggctt cccatacaat cgatagattg 1260
   tcgcacctga ttgcccgaca ttatcgcgag cccatttata cccatataaa tcagcatcca 1320
   tgttggaatt taatcgcggc ctagagcaag acgtttcccg ttgaatatgg ctcataacac 1380
   cccttgtatt actgtttatg taagcagaca gttttattgt tcatgaccaa aatcccttaa 1440
   cgtgagtttt cgttccactg agcgtcagac cccgtagaaa agatcaaagg atcttcttga 1500
   gatccttttt ttctgcgcgt aatctgctgc ttgcaaacaa aaaaaccacc gctaccagcg 1560
   gtggtttgtt tgccggatca agagctacca actctttttc cgaaggtaac tggcttcagc 1620
   agagcgcaga taccaaatac tgtccttcta gtgtagccgt agttaggcca ccacttcaag 1680
   aactctgtag caccgcctac atacctcgct ctgctaatcc tgttaccagt ggctgctgcc 1740
   agtggcgata agtcgtgtct taccgggttg gactcaagac gatagttacc ggataaggcg 1800
   cagcggtcgg gctgaacggg gggttcgtgc acacagccca gcttggagcg aacgacctac 1860
   accgaactga gatacctaca gcgtgagcta tgagaaagcg ccacgcttcc cgaagggaga 1920
   aaggcggaca ggtatccggt aagcggcagg gtcggaacag gagagcgcac gagggagctt 1980
   ccagggggaa acgcctggta tctttatagt cctgtcgggt ttcgccacct ctgacttgag 2040
   cgtcgatttt tgtgatgctc gtcagggggg cggagcctat ggaaaaacgc cagcaacgcg 2100
   gcctttttac ggttcctggc cttttgctgg ccttttgctc acatgttctt tcctgcgtta 2160
   tcccctgatt ctgtggataa ccgtattacc gcctttgagt gagctgatac cgctcgccgc 2220
   agccgaacga ccgagcgcag cgagtcagtg agcgaggaag cggaagagcg cctgatgcgg 2280
   tattttctcc ttacgcatct gtgcggtatt tcacaccgca tatatggtgc actctcagta 2340
   caatctgctc tgatgccgca tagttaagcc agtatacact ccgctatcgc tacgtgactg 2400
   ggtcatggct gcgccccgac acccgccaac acccgctgac gcgccctgac gggcttgtct 2460
   gctcccggca tccgcttaca gacaagctgt gaccgtctcc gggagctgca tgtgtcagag 2520
   gttttcaccg tcatcaccga aacgcgcgag gcagctgcgg taaagctcat cagcgtggtc 2580
   gtgaagcgat tcacagatgt ctgcctgttc atccgcgtcc agctcgttga gtttctccag 2640
   aagcgttaat gtctggcttc tgataaagcg ggccatgtta agggcggttt tttcctgttt 2700
   ggtcactgat gcctccgtgt aagggggatt tctgttcatg ggggtaatga taccgatgaa 2760
   acgagagagg atgctcacga tacgggttac tgatgatgaa catgcccggt tactggaacg 2820
   ttgtgagggt aaacaactgg cggtatggat gcggcgggac cagagaaaaa tcactcaggg 2880
   tcaatgccag cgcttcgtta atacagatgt aggtgttcca cagggtagcc agcagcatcc 2940
   tgcgatgcag atccggaaca taatggtgca gggcgctgac ttccgcgttt ccagacttta 3000
   cgaaacacgg aaaccgaaga ccattcatgt tgttgctcag gtcgcagacg ttttgcagca 3060
   gcagtcgctt cacgttcgct cgcgtatcgg tgattcattc tgctaaccag taaggcaacc 3120
   ccgccagcct agccgggtcc tcaacgacag gagcacgatc atgcgcaccc gtggggccgc 3180
   catgccggcg ataatggcct gcttctcgcc gaaacgtttg gtggcgggac cagtgacgaa 3240
   ggcttgagcg agggcgtgca agattccgaa taccgcaagc gacaggccga tcatcgtcgc 3300
   gctccagcga aagcggtcct cgccgaaaat gacccagagc gctgccggca cctgtcctac 3360
   gagttgcatg ataaagaaga cagtcataag tgcggcgacg atagtcatgc cccgcgccca 3420
   ccggaaggag ctgactgggt tgaaggctct caagggcatc ggtcgagatc ccggtgccta 3480
   atgagtgagc taacttacat taattgcgtt gcgctcactg cccgctttcc agtcgggaaa 3540
   cctgtcgtgc cagctgcatt aatgaatcgg ccaacgcgcg gggagaggcg gtttgcgtat 3600
   tgggcgccag ggtggttttt cttttcacca gtgagacggg caacagctga ttgcccttca 3660
   ccgcctggcc ctgagagagt tgcagcaagc ggtccacgct ggtttgcccc agcaggcgaa 3720
   aatcctgttt gatggtggtt aacggcggga tataacatga gctgtcttcg gtatcgtcgt 3780
   atcccactac cgagatatcc gcaccaacgc gcagcccgga ctcggtaatg gcgcgcattg 3840
   cgcccagcgc catctgatcg ttggcaacca gcatcgcagt gggaacgatg ccctcattca 3900
   gcatttgcat ggtttgttga aaaccggaca tggcactcca gtcgccttcc cgttccgcta 3960
   tcggctgaat ttgattgcga gtgagatatt tatgccagcc agccagacgc agacgcgccg 4020
   agacagaact taatgggccc gctaacagcg cgatttgctg gtgacccaat gcgaccagat 4080
   gctccacgcc cagtcgcgta ccgtcttcat gggagaaaat aatactgttg atgggtgtct 4140
   ggtcagagac atcaagaaat aacgccggaa cattagtgca ggcagcttcc acagcaatgg 4200
   catcctggtc atccagcgga tagttaatga tcagcccact gacgcgttgc gcgagaagat 4260
   tgtgcaccgc cgctttacag gcttcgacgc cgcttcgttc taccatcgac accaccacgc 4320
   tggcacccag ttgatcggcg cgagatttaa tcgccgcgac aatttgcgac ggcgcgtgca 4380
   gggccagact ggaggtggca acgccaatca gcaacgactg tttgcccgcc agttgttgtg 4440
   ccacgcggtt gggaatgtaa ttcagctccg ccatcgccgc ttccactttt tcccgcgttt 4500
   tcgcagaaac gtggctggcc tggttcacca cgcgggaaac ggtctgataa gagacaccgg 4560
   catactctgc gacatcgtat aacgttactg gtttcacatt caccaccctg aattgactct 4620
   cttccgggcg ctatcatgcc ataccgcgaa aggttttgcg ccattcgatg gtgtccggga 4680
   tctcgacgct ctcccttatg cgactcctgc attaggaagc agcccagtag taggttgagg 4740
   ccgttgagca ccgccgccgc aaggaatggt gcatgcaagg agatggcgcc caacagtccc 4800
   ccggccacgg ggcctgccac catacccacg ccgaaacaag cgctcatgag cccgaagtgg 4860
   cgagcccgat cttccccatc ggtgatgtcg gcgatatagg cgccagcaac cgcacctgtg 4920
   gcgccggtga tgccggccac gatgcgtccg gcgtagagga tcgagatctc gatcccgcga 4980
   aattaatacg actcactata ggggaattgt gagcggataa caattcccct ctagaaataa 5040
   ttttgtttaa ctttaagaag gagatatacc atgggcagca gccatcatca tcatcatcac 5100
   agcagcggcc tggtgccgcg cggcagccat atggaaggtg aatatattaa actcaaagtc 5160
   attggacagg atagcagtga gattcacttc aaagtgaaaa tgacaacaca tctcaagaaa 5220
   ctcaaagaat catactgtca aagacagggt gttccaatga attcactcag gtttctcttt 5280
   gagggtcaga gaattgctga taatcatact ccaaaagaac tgggaatgga ggaagaagat 5340
   gtgattgaag tctaccagga acaaaccggt ggatccatgc aggtgaaact ggaacagagc 5400
   ggcgcgggcg tggtgaaacc gggcgcgagc gtgaaactga gctgcaaagc gagcggcttt 5460
   aacattaaag atagctatat gcattggctg cgccagggcc cgggccagcg cctggaatgg 5520
   attggctgga ttgatccgga aaacggcgat accgaatatg cgccgaaatt tcagggcaaa 5580
   gcgaccttta ccaccgatac cagcgcgaac accgcgtatc tgggcctgag cagcctgcgc 5640
   ccggaagata ccgcggtgta ttattgcaac gaaggcaccc cgaccggccc gtattatttt 5700
   gattattggg gccagggcac cctggtgacc gtgagcagcg gcggcggcgg cagcggcggc 5760
   ggcggcagcg gcggcggcgg cagcgaaaac gtgctgaccc agagcccgag cagcatgagc 5820
   gtgagcgtgg gcgatcgcgt gaccattgcg tgcagcgcga gcagcagcgt gccgtatatg 5880
   cattggctgc agcagaaacc gggcaaaagc ccgaaactgc tgatttatct gaccagcaac 5940
   ctggcgagcg gcgtgccgag ccgctttagc ggcagcggca gcggcaccga ttatagcctg 6000
   accattagca gcgtgcagcc ggaagatgcg gcgacctatt attgccagca gcgcagcagc 6060
   tatccgctga cctttggcgg cggcaccaaa ctggaaatta aagcggcggc gggcagcgcg 6120
   agcggcggcg gcggcagcgg cggcggcact agtggcggcg gcggcagcgc ggagggcggc 6180
   agcctggccg cgctgaccgc gcaccaggcc tgccacctgc cgctggagac cttcacccgt 6240
   catcgccagc cgcgcggctg ggaacaactg gagcagtgcg gctatccggt gcagcggctg 6300
   gtcgccctct acctggcggc gcggctgtcg tggaaccagg tcgaccaggt gatccgcaac 6360
   gccctggcca gccccggcag cggcggcgac ctgggcgaag cgatccgcga gcagccggag 6420
   caggcccgtc tggccctgac cctggccgcc gccgagagcg agcgcttcgt ccggcagggc 6480
   acaggcaacg acgaggccgg cgcggccagc gccgacgtgg tgagcctgac ctgcccggtc 6540
   gccgccggtg aatgcgcggg cccggcggac agcggcgacg ccctgctgga gcgcaactat 6600
   cccactggcg cggagttcct cggcgacggc ggcgacatca gcttcagcac ccgcggcacg 6660
   cagaactgga cggtggagcg gctgctccag gcgcaccgcc aactggagga gcgcggctat 6720
   gtgttcgtcg gctaccacgg caccttcctc gaagcggcgc aaagcatcgt cttcggcggg 6780
   gtgcgcgcgc gcagccagga cctcgacgcg atctggcgcg gtttctatat cgccggcgat 6840
   ccggcgctgg cctacggcta cgcccaggac caggaacccg acgcgcgcgg ccggatccgc 6900
   aacggtgccc tgctgcgggt ctatgtgccg cgctcgagtc tgccgggctt ctaccgcacc 6960
   ggcctgaccc tggccgcgcc ggaggcggcg ggcgaggtcg aacggctgat cggccatccg 7020
   ctgccgctgc gcctggacgc catcaccggc cccgaggagg aaggcgggcg cctggagacc 7080
   attctcggct ggccgctggc cgagcgcacc gtggtgattc cctcggcgat ccccaccgac 7140
   ccgcgcaacg tcggcggcga cctcgacccg tccagcatcc ccgacaagga acaggcgatc 7200
   agcgccctgc cggactacgc cagccagccc ggcaaaccgc cgcgcgagga cctgaagtga 7260
   ctcgagcacc accaccacca ccactgagat ccggctgcta acaaagcccg aaaggaagct 7320
   gagttggctg ctgccaccgc tgagcaataa ctagcataac cccttggggc ctctaaacgg 7380
   gtcttgaggg gttttttgct gaaaggagga actatatccg gat 7423
<210> 12
   <211> 306
   <212> DNA
   <213> Artificial Sequence
<220>
   <223> Sumo-tag DNA sequence encoded by the PEantiC construct (PEantiCP28S)
<400> 12
   atgggcagca gccatcatca tcatcatcac agcagcggcc tggtgccgcg cggcagccat 60
   atggaaggtg aatatattaa actcaaagtc attggacagg atagcagtga gattcacttc 120
   aaagtgaaaa tgacaacaca tctcaagaaa ctcaaagaat catactgtca aagacagggt 180
   gttccaatga attcactcag gtttctcttt gagggtcaga gaattgctga taatcatact 240
   ccaaaagaac tgggaatgga ggaagaagat gtgattgaag tctaccagga acaaaccggt 300
   ggatcc 306
<210> 13
   <211> 741
   <212> DNA
   <213> Artificial Sequence
<220>
   <223> DNA sequence of Anti-CEA ScFv encoded by the the PEantiC construct (PEantiCP28S)
<400> 13
   atgcaggtga aactggaaca gagcggcgcg ggcgtggtga aaccgggcgc gagcgtgaaa 60
   ctgagctgca aagcgagcgg ctttaacatt aaagatagct atatgcattg gctgcgccag 120
   ggcccgggcc agcgcctgga atggattggc tggattgatc cggaaaacgg cgataccgaa 180
   tatgcgccga aatttcaggg caaagcgacc tttaccaccg ataccagcgc gaacaccgcg 240
   tatctgggcc tgagcagcct gcgcccggaa gataccgcgg tgtattattg caacgaaggc 300
   accccgaccg gcccgtatta ttttgattat tggggccagg gcaccctggt gaccgtgagc 360
   agcggcggcg gcggcagcgg cggcggcggc agcggcggcg gcggcagcga aaacgtgctg 420
   acccagagcc cgagcagcat gagcgtgagc gtgggcgatc gcgtgaccat tgcgtgcagc 480
   gcgagcagca gcgtgccgta tatgcattgg ctgcagcaga aaccgggcaa aagcccgaaa 540
   ctgctgattt atctgaccag caacctggcg agcggcgtgc cgagccgctt tagcggcagc 600
   ggcagcggca ccgattatag cctgaccatt agcagcgtgc agccggaaga tgcggcgacc 660
   tattattgcc agcagcgcag cagctatccg ctgacctttg gcggcggcac caaactggaa 720
   attaaagcgg cggcgggcag c 741
<210> 14
   <211> 54
   <212> DNA
   <213> Artificial Sequence
<220>
   <223> DNA sequence of linker between anti-CEA ScFv and Exotoxin A fragment as encoded by the PEantiC construct (PEantiCP28S)
<400> 14
   gcgagcggcg gcggcggcag cggcggcggc actagtggcg gcggcggcag cgcg 54
<210> 15
   <211> 1089
   <212> DNA
   <213> Artificial Sequence
<220>
   <223> Exotoxin A fragment sequence as encoded by the the PEantiC construct (PEantiCP28S)
<400> 15
   gagggcggca gcctggccgc gctgaccgcg caccaggcct gccacctgcc gctggagacc      60
   ttcacccgtc atcgccagcc gcgcggctgg gaacaactgg agcagtgcgg ctatccggtg     120
   cagcggctgg tcgccctcta cctggcggcg cggctgtcgt ggaaccaggt cgaccaggtg     180
   atccgcaacg ccctggccag ccccggcagc ggcggcgacc tgggcgaagc gatccgcgag     240
   cagccggagc aggcccgtct ggccctgacc ctggccgccg ccgagagcga gcgcttcgtc     300
   cggcagggca caggcaacga cgaggccggc gcggccagcg ccgacgtggt gagcctgacc     360
   tgcccggtcg ccgccggtga atgcgcgggc ccggcggaca gcggcgacgc cctgctggag     420
   cgcaactatc ccactggcgc ggagttcctc ggcgacggcg gcgacatcag cttcagcacc     480
   cgcggcacgc agaactggac ggtggagcgg ctgctccagg cgcaccgcca actggaggag     540
   cgcggctatg tgttcgtcgg ctaccacggc accttcctcg aagcggcgca aagcatcgtc     600
   ttcggcgggg tgcgcgcgcg cagccaggac ctcgacgcga tctggcgcgg tttctatatc     660
   gccggcgatc cggcgctggc ctacggctac gcccaggacc aggaacccga cgcgcgcggc     720
   cggatccgca acggtgccct gctgcgggtc tatgtgccgc gctcgagtct gccgggcttc     780
   taccgcaccg gcctgaccct ggccgcgccg gaggcggcgg gcgaggtcga acggctgatc     840
   ggccatccgc tgccgctgcg cctggacgcc atcaccggcc ccgaggagga aggcgggcgc     900
   ctggagacca ttctcggctg gccgctggcc gagcgcaccg tggtgattcc ctcggcgatc     960
   cccaccgacc cgcgcaacgt cggcggcgac ctcgacccgt ccagcatccc cgacaaggaa    1020
   caggcgatca gcgccctgcc ggactacgcc agccagcccg gcaaaccgcc gcgcgaggac    1080
   ctgaagtga                                                            1089
<210> 16
   <211> 1884
   <212> DNA
   <213> Artificial Sequence
<220>
   <223> DNA sequence of the PEantiC immunotoxin: the anti-CEA scFv linked via a linker to a fragment of Exotoxin A (Domains II, IB and III), as encoded by the PEantiC construct (PEantiCP28S).
<400> 16
   atgcaggtga aactggaaca gagcggcgcg ggcgtggtga aaccgggcgc gagcgtgaaa 60
   ctgagctgca aagcgagcgg ctttaacatt aaagatagct atatgcattg gctgcgccag 120
   ggcccgggcc agcgcctgga atggattggc tggattgatc cggaaaacgg cgataccgaa 180
   tatgcgccga aatttcaggg caaagcgacc tttaccaccg ataccagcgc gaacaccgcg 240
   tatctgggcc tgagcagcct gcgcccggaa gataccgcgg tgtattattg caacgaaggc 300
   accccgaccg gcccgtatta ttttgattat tggggccagg gcaccctggt gaccgtgagc 360
   agcggcggcg gcggcagcgg cggcggcggc agcggcggcg gcggcagcga aaacgtgctg 420
   acccagagcc cgagcagcat gagcgtgagc gtgggcgatc gcgtgaccat tgcgtgcagc 480
   gcgagcagca gcgtgccgta tatgcattgg ctgcagcaga aaccgggcaa aagcccgaaa 540
   ctgctgattt atctgaccag caacctggcg agcggcgtgc cgagccgctt tagcggcagc 600
   ggcagcggca ccgattatag cctgaccatt agcagcgtgc agccggaaga tgcggcgacc 660
   tattattgcc agcagcgcag cagctatccg ctgacctttg gcggcggcac caaactggaa 720
   attaaagcgg cggcgggcag cgcgagcggc ggcggcggca gcggcggcgg cactagtggc 780
   ggcggcggca gcgcggaggg cggcagcctg gccgcgctga ccgcgcacca ggcctgccac 840
   ctgccgctgg agaccttcac ccgtcatcgc cagccgcgcg gctgggaaca actggagcag 900
   tgcggctatc cggtgcagcg gctggtcgcc ctctacctgg cggcgcggct gtcgtggaac 960
   caggtcgacc aggtgatccg caacgccctg gccagccccg gcagcggcgg cgacctgggc 1020
   gaagcgatcc gcgagcagcc ggagcaggcc cgtctggccc tgaccctggc cgccgccgag 1080
   agcgagcgct tcgtccggca gggcacaggc aacgacgagg ccggcgcggc cagcgccgac 1140
   gtggtgagcc tgacctgccc ggtcgccgcc ggtgaatgcg cgggcccggc ggacagcggc 1200
   gacgccctgc tggagcgcaa ctatcccact ggcgcggagt tcctcggcga cggcggcgac 1260
   atcagcttca gcacccgcgg cacgcagaac tggacggtgg agcggctgct ccaggcgcac 1320
   cgccaactgg aggagcgcgg ctatgtgttc gtcggctacc acggcacctt cctcgaagcg 1380
   gcgcaaagca tcgtcttcgg cggggtgcgc gcgcgcagcc aggacctcga cgcgatctgg 1440
   cgcggtttct atatcgccgg cgatccggcg ctggcctacg gctacgccca ggaccaggaa 1500
   cccgacgcgc gcggccggat ccgcaacggt gccctgctgc gggtctatgt gccgcgctcg 1560
   agtctgccgg gcttctaccg caccggcctg accctggccg cgccggaggc ggcgggcgag     1620
   gtcgaacggc tgatcggcca tccgctgccg ctgcgcctgg acgccatcac cggccccgag     1680
   gaggaaggcg ggcgcctgga gaccattctc ggctggccgc tggccgagcg caccgtggtg     1740
   attccctcgg cgatccccac cgacccgcgc aacgtcggcg gcgacctcga cccgtccagc     1800
   atccccgaca aggaacaggc gatcagcgcc ctgccggact acgccagcca gcccggcaaa     1860
   ccgccgcgcg aggacctgaa gtga                                           1884

## Description

### Field of the invention

The invention relates to methods for purification of proteins comprising wild type Exotoxin A, Exotoxin A muteins and fragments of Exotoxin A, in particular active proteins comprising the cytotoxic domain III of Exotoxin A. The method allows for protein purification from both soluble fraction and inclusion bodies and does not require the use of high concentrations of chaotropic agents.

### Background to the Invention

Most of the systems designed for protein overexpression in bacterial hosts are focused on production of protein in soluble form. Proteins obtained in the soluble fraction are easy to purify and usually retain their activity. However, high-level expression of recombinant proteins often leads to a formation of insoluble aggregates, commonly referred to as inclusion bodies (IBs) [Zhu, S., Gong, C., Ren, L., Li, X., Song, D., Zheng, G. (2013). A simple and effective strategy for solving the problem of inclusion bodies in recombinant protein technology: His-tag deletions enhance soluble expression. Appl Microbiol Biotechnol 97, 837-845]. IBs are cytoplasmic structures thought to be formed when the equilibrium between soluble protein and aggregated protein is unbalanced. Thus, IBs are usually formed when the host's protein translation system is overloaded. It has been reported that formation of IBs is not only reversible *in vivo*, but also that proteins accumulated in IBs retain their native-like secondary structures. Therefore, the process of protein recovery from IBs without disturbing their native conformation would be beneficial for their activity. Accordingly, IBs can be a very valuable source of protein for purification. Moreover, these aggregates are resistant to proteolytic degradation and usually contain pure proteins [Carrio, M.M., Villaverde, A. (2001) Protein aggregation as bacterial inclusion bodies is reversible. FEBS Lett 489, 29-33; Singh, S.M., Panda, A.K. (2005) Solubilization and refolding of bacterial inclusion body proteins. J Biosci Bioeng 99, 303-310; Yamaguchi, H., Miyazaki, M. (2014) Refolding techniques for recovering biologically active recombinant proteins from inclusion bodies. Biomolecules 4, 235-251; Peternel, S., Komel, R. (2011) Active protein aggregates produced in Escherichia coli. Int J Mol Sci 12, 8275-8287; Ventura, S., Villaverde, A. (2006) Protein quality in bacterial inclusion bodies. Trends Biotechnol 24, 179-185]. Proteins overexpressed as IBs can be solubilized using high concentrations of chaotropic agents, such as urea or guanidine hydrochloride; detergents such as SDS, or acetonitrile/propanol [Palmer, I., Wingfield, P.T. (2012) Preparation and extraction of insoluble (inclusion-body) proteins from Escherichia coli. Curr Protoc Protein Sci Chapter 6, Unit 6 3; Schlager, B., Straessle, A., Hafen, E. (2012) Use of anionic denaturing detergents to purify insoluble proteins after overexpression. BMC Biotechnol 12, 95; Tsumoto, K., Abe, R., Ejima, D., Arakawa, T. (2010) Non-denaturing solubilization of inclusion bodies. Curr Pharm Biotechnol 11, 309-312]. The proteins solubilized in the aforementioned way are usually fully unfolded. Refolding requires removal of the denaturing agent, usually by dialysis to buffers providing appropriate redox conditions. The refolding step is a very cumbersome process, requiring large volumes of buffers and laborious work [Yamaguchi, H., Miyazaki, M. (2014) Refolding techniques for recovering biologically active recombinant proteins from inclusion bodies. Biomolecules 4, 235-251]. Newly emerging methods use very mild techniques of solubilization, which do not interfere with protein structure within IBs. For instance, human growth hormone (hGH) was successfully purified from aggregates using low concentrations of guanidine hydrochloride and Triton™ X-100 followed by high pressure homogenization [Bailey, S.M., Blum, P.H., Meagher, M.M. (1995) Improved homogenization of recombinant Escherichia coli following pretreatment with guanidine hydrochloride. Biotechnol Prog 11, 533-539]. Human growth hormone was also successfully purified using a high pH (12.5) along with low concentration of urea (2M) [Singh, S.M., Panda, A.K. (2005) Solubilization and refolding of bacterial inclusion body proteins. J Biosci Bioeng 99, 303-310]. Glutathione S-transferase was recovered from IBs using a combination of 10% sarkosyl, Triton™ X-100 and CHAPS [Tao, H., Liu, W., Simmons, B.N., Harris, H.K., Cox, T.C., Massiah, M.A. (2010) Purifying natively folded proteins from inclusion bodies using sarkosyl, Triton™ X-100, and CHAPS. Biotechniques 48, 61-64]. L-asparaginase-II was also expressed in *E. coli* in the form of IBs. The protein was solubilized with the aid of 4M urea [Upadhyay, A.K., Singh, A., Mukherjee, K.J., Panda, A.K. (2014) Refolding and purification of recombinant L-asparaginase from inclusion bodies of E. coli into active tetrameric protein. Front Microbiol 5, 486].

Full length Exotoxin A and a C-terminal fragment of Exotoxin A (domain III) were expressed in *E. coli* strain 294 but were not purified [Gray, G.L., Smith, D.H., Baldridge, J.S., Harkins, R.N., Vasil, M.L., Chen, E.Y., Heyneker, H.L. (1984) Cloning, nucleotide sequence, and expression in Escherichia coli of the exotoxin A structural gene of Pseudomonas aeruginosa. Proc Natl Acad Sci U S A 81, 2645-2649].

Full length Exotoxin A and truncated Exotoxin A (lacking binding domain) were expressed in *E. coli* BL21(DE3) and purified from soluble fraction [Behzad, B., Safar, F., Jafar, M., Yadollah, O., Nazali, S. (2013) Recombinant Expression and Purification of Pseudomonas aeruginosa Truncated Exotoxin A in Escherichia coli Pharmaceutical Sciences 19(1), 4].

To investigate the role of amino acids exposed on the surface of domain III, 15 amino acids were replaced and thus 29 different protein mutants were generated. Mutants were fused to OmpA protein and expressed in *E. coli* BL21(DE3) in the periplasm. The soluble protein was purified directly from fermentation broth [Benhar, I., Wang, Q.C., FitzGerald, D., Pastan, I. (1994) Pseudomonas exotoxin A mutants. Replacement of surface-exposed residues in domain III with cysteine residues that can be modified with polyethylene glycol in a site-specific manner. J Biol Chem 269, 13398-13404].

Exotoxin A mutein with domain II replaced by barnase protein (the extracellular ribonuclease of *Bacillus amyloliquefaciens*) was constructed to study the translocation process of Exotoxin A. The mutein was fused to OmpA protein and expressed in *E. coli* BL21(DE3) in the periplasm. For purification, the protein was denatured in 7M guanidine hydrochloride, 5mM EDTA and 100mM Tris-HCI, pH 8.0, and refolded afterwards [Prior, T.I., FitzGerald, D.J., Pastan, I. (1992) Translocation mediated by domain II of Pseudomonas exotoxin A: transport of barnase into the cytosol. Biochemistry 31, 3555-3559].

Exotoxin A was fused to proteins with different unfolding abilities. Such chimeras were used to study endosomal translocation of the toxin. Exotoxin A and its chimeras were fused to OmpA protein and expressed in *E. coli* BL21(DE3) in the periplasm [Morlon-Guyot, J., Mere, J., Bonhoure, A., Beaumelle, B. (2009) Processing of Pseudomonas aeruginosa exotoxin A is dispensable for cell intoxication. Infect Immun 77, 3090-3099].

IL6(T23)-PE38KDEL is a chimeric molecule composed of an N-terminally truncated interleukin 6 (IL6), missing the N-terminal 23 amino acids, fused to a truncated mutant form of *Pseudomonas* exotoxin (PE38KDEL). This Exotoxin A-based immunotoxin has been successfully purified from IBs using 6M Gu-HCl after expression in BL21(DE3) [Guo, D.J., Han, J.S., Li, Y.S., Liu, Z.S., Lu, S.Y., Ren, H.L. (2012) In vitro and in vivo antitumor effects of the recombinant immunotoxin IL6(T23)-PE38KDEL in multiple myeloma. Oncol Lett 4, 311-318].

scFv(MUC1)-ETA immunotoxin was constructed by fusion of truncated *Pseudomonas aeruginosa* exotoxin A (ETA) to a high-affinity binder, phage-derived scFv MUC1. The immunotoxin was expressed in *E. coli* strain CC118 and purified from the soluble fraction [Singh, R., Samant, U., Hyland, S., Chaudhari, P.R., Wels, W.S., Bandyopadhyay, D. (2007) Target-specific cytotoxic activity of recombinant immunotoxin scFv(MUC1)-ETA on breast carcinoma cells and primary breast tumors. Mol Cancer Ther 6, 562-569].

The murine anti-CD30 hybridoma Ki-4 was used to construct a new recombinant immunotoxin (rIT) for possible clinical use in patients with CD30(+) lymphoma. Functional Ki-4 scFv obtained by phage display and selection of binding on the CD30-expressing Hodgkin's lymphoma cell line L540cy was inserted into the bacterial expression vector pBM1.1 and fused to a deletion mutant of *Pseudomonas* exotoxin A (ETA'). The immunotoxin was expressed in *E. coli* BL21(DE3) in the periplasm [Barth, S., Huhn, M., Matthey, B., Tawadros, S., Schnell, R., Schinkothe, T., Diehl, V., Engert, A. (2000) Ki-4(scFv)-ETA', a new recombinant anti-CD30 immunotoxin with highly specific cytotoxic activity against disseminated Hodgkin tumors in SCID mice. Blood 95, 3909-3914].

To improve specificity of rITs, the original cell-binding domain of ETA was exchanged with a functional humanized single chain variable fragment (hscFv) anti-CML antibody. The immunotoxin was expressed in *E. coli* BL21(DE3) in the periplasm [Zhu, X., Tao, K., Li, Y., Li, S., Zhang, L., Wang, D., Zhong, L., Feng, W. (2013) A new recombinant immunotoxin hscFv-ETA' demonstrates specific cytotoxicity against chronic myeloid leukemia cells in vitro. Immunol Lett 154, 18-24].

Recombinant immunotoxin H22(scFv)-ETA' comprising a truncated *Pseudomonas* exotoxin A (PE) and a humanized scFv antibody against CD64 was expressed in the periplasm in BL21(DE3) [Tur, M.K., Huhn, M., Jost, E., Thepen, T., Brummendorf, T.H., Barth, S. (2011) In vivo efficacy of the recombinant anti-CD64 immunotoxin H22(scFv)-ETA' in a human acute myeloid leukemia xenograft tumor model. Int J Cancer 129, 1277-1282; Tur, M.K., Huhn, M., Thepen, T., Stocker, M., Krohn, R., Vogel, S., Jost, E., Osieka, R., van de Winkel, J.G., Fischer, R. et al. (2003) Recombinant CD64-specific single chain immunotoxin exhibits specific cytotoxicity against acute myeloid leukemia cells. Cancer Res 63, 8414-8419].

HM1.24-ETA' was generated by genetic fusion of a CD317-specific single-chain Fv (scFv) antibody and a truncated variant of *Pseudomonas aeruginosa* exotoxin A (ETA'). The immunotoxin was expressed in Arctic Express (DE3) in the periplasm [Staudinger, M., Glorius, P., Burger, R., Kellner, C., Klausz, K., Gunther, A., Repp, R., Klapper, W., Gramatzki, M., Peipp, M. (2014) The novel immunotoxin HM1.24-ETA' induces apoptosis in multiple myeloma cells. Blood Cancer J 4, e219].

The monoclonal antibody (mAb) B3 binds to many human cancer cells. The Fv moiety of B3 was connected to a truncated form of *Pseudomonas* exotoxin A (PE; PE38KDEL). During expression the immunotoxin accumulated in cytoplasmic IBs. The protein was denatured, purified, reduced and diluted into redox shuffling refolding buffer [Reiter, Y., Brinkmann, U., Webber, K.O., Jung, S.H., Lee, B., Pastan, I. (1994) Engineering interchain disulfide bonds into conserved framework regions of Fv fragments: improved biochemical characteristics of recombinant immunotoxins containing disulfide-stabilized Fv. Protein Eng 7, 697-704].

A divalent immunotoxin, [B3(FabH1)-PE38]2, was constructed by combining the Fab domain of the B3 antibody as the cell targeting domain and *Pseudomonas* exotoxin A (PE) as the cytotoxic domain. Monoclonal antibody B3 is the murine antibody (lgG1) directed against LewisY-related carbohydrate antigens, which are abundant on the surface of many carcinomas. The Fc region of B3 antibody was substituted with the truncated form of PE (38 kDa, PE38) at the DNA level. Each polypeptide for the recombinant immunotoxins was overexpressed in *E. coli* and collected as IBs. Each IB was solubilized by denaturation and refolded [Park, J.H., Kwon, H.W., Chung, H.K., Kim, I.H., Ahn, K., Choi, E.J., Pastan, I., Choe, M. (2001) A divalent recombinant immunotoxin formed by a disulfide bond between the extension peptide chains. Mol Cells 12, 398-402].

The solubilization methods described thus far are efficient, however, it is not possible to predict which purification methods will work for a particular protein. Moreover, purification from IBs is sometimes the only possible way to obtain the protein of interest.

Human growth hormone (r-hGH) was purified from IBs obtained from *E. coli* strain M15 cells. Pure IBs were solubilized in the presence of 2 M urea solution at pH 12. [Singh, S.M., Panda, A.K. (2005) Solubilization and refolding of bacterial inclusion body proteins. J Biosci Bioeng 99, 303-310]. In the method described, mechanical disruption of the cells was achieved using a French press. Inclusion bodies were isolated by a long, expensive and difficult process involving isolation by centrifugation and subsequent high-speed centrifugation in sucrose gradient. For high scale purification IB were extensively washed with detergents (Tris buffers containing EDTA, Deoxycholate, using overnight incubations) and washed with water to remove all detergents. To prepare for the purification step, IBs were solubilized in buffer containing 100mMTris, 2M urea and pH12.5 this was followed by dilution with buffer: 50mM Tris-HCI, 0,5mM EDTA, 2M urea, 10% glycerol, 5% sucrose, 1mM PMSF, pH8,0, which did not decrease the urea concentration, but did decrease the pH. Purification was performed by DEAE (anion exchange chromatography).

A fragment corresponding to bonnet monkey (*Macaca radiata*) zona pellucida glycoprotein-C (bmZPC), excluding the N-terminal signal sequence and the C-terminal transmembrane-like domain was expressed as IBs in BL21(DE3). Protein was solubilized in buffer at pH 12.0 containing 2M urea [Patra, A.K., Gahlay, G.K., Reddy, B.V., Gupta, S.K., Panda, A.K. (2000) Refolding, structural transition and spermatozoa-binding of recombinant bonnet monkey (Macaca radiata) zona pellucida glycoprotein-C expressed in Escherichia coli. Eur J Biochem 267, 7075-7081]. In the method described, mechanical disruption of the cells was achieved using sonication and a French press. Inclusion bodies were isolated by a long, expensive and difficult process involving isolation by centrifugation and subsequent high-speed centrifugation in sucrose gradient. For high scale purification, IB were extensively washed with detergents (Tris buffers containing EDTA a Deoxycholate, using overnight incubations) and washed with water to remove all detergents. To prepare for the purification step, IBs were solubilized in buffer containing 100mMTris, 2M urea and pH12.5; this was followed by dilution with MQ water, which decreased the urea concentration and addition of HCI which decreased the pH. Purification was performed by DEAE (anion exchange chromatography), followed by dialysis to change the buffer and then lyophilization of the protein fragment obtained.

Exotoxin A, a three-domain protein produced by *Pseudomonas aeruginosa,* is one of the most investigated toxins used in targeted cancer therapy [Weldon, J.E., Pastan, I. (2011) A guide to taming a toxin-recombinant immunotoxins constructed from Pseudomonas exotoxin A for the treatment of cancer. FEBS J 278, 4683-4700; Wolf, P., Elsasser-Beile, U. (2009) Pseudomonas exotoxin A: from virulence factor to anti-cancer agent. Int J Med Microbiol 299, 161-176]. Consequently, numerous Exotoxin A-based immunotoxins are in clinical trials as anticancer drugs [Alewine, C., Hassan, R., Pastan, I. (2015) Advances in anticancer immunotoxin therapy. The Oncologist 20, 176-185]. It has been reported that Exotoxin A may be produced and purified from both soluble and insoluble fractions. It has also been reported that low concentrations of urea have a beneficial effect on the toxin [Beattie, B.K., Merrill, A.R. (1996) In vitro enzyme activation and folded stability of Pseudomonas aeruginosa exotoxin A and its C-terminal peptide. Biochemistry 35, 9042-9051].

Document US2009/203884 discloses the purification of recombinant Exotoxin A - gp120 fusion protein. The method is based on solublization of protein from isolated and intensively washed inclusion bodies but not from a pure bacterial pellet.

Documents WO2006/001023 and Wang et al. 2008 ("Expression, purification and characterization of an immunotoxin containing a humanized anti-CD25 single chain fragment variable antibody fused to a modified truncated Pseudomonas exotoxin A", Protein Expression and Purification, Academic press, San Diego, CA, Vol. 58, no. 1 (2008-01-28)) describe method of solubilizing the isolated inclusion bodies using 8M urea solution. However, 8M urea leads to complete denaturation of protein that necessitates also a proper process of protein refolding after or prior purification.

Document Anupam Singh et al. 2015 : (Protein recovery from inclusion bodies of Escherichia coli using mild solubilization process", Microbal Cell Factories, Biomed Central, Vol. 14, no. 1, 25-03-2015), is a review about protein recovery from inclusion bodies of E. coli., which describe the general idea of using buffers with high pH (>12) in combination with 2M urea for solubilization of inclusion bodies. The described methods require purification of inclusion bodies and refolding of the solubilized protein.

It is an object of the invention to provide a method for purifying a protein comprising an Exotoxin A protein, which can be Exotoxin A, an Exotoxin A mutein or an Exotoxin A fragment, using a mildly denaturing urea solution at alkaline pH. Another object of the invention is to provide a method for protein purification whereby the purified protein comprising an Exotoxin A protein maintains cytotoxic activity or shows an improvement in cytotoxic activity relative to cytotoxin isolated from soluble fraction. A further object of the invention is also to provide a protocol for simple and fast purification of recombinant proteins, comprising an active Exotoxin A protein, with great purity.

### Statements of Invention

The invention provides a method for purifying a protein comprising an Exotoxin A, Exotoxin A mutein or active fragment thereof, the method comprising:
(a) solubilizing a sample comprising the protein in a solubilization buffer containing urea and at alkaline pH, the protein is a recombinant protein, sample is a bacterial pellet obtained following expression of the recombinant protein in bacteria, wherein the solubilization buffer contains urea at a concentration in the range of from 1.8 to 2.2M and has a buffer pH in the range of from 11.5 to 12.5, and wherein the solubilization buffer is a phosphate buffer,
(b) decreasing the concentration of urea and the pH by dilution using a dilution buffer, prior to purification of the protein, and wherein the dilution buffer comprises phosphate buffer without urea at a pH in the range of from pH 7.5 to 8.5, preferably wherein three volumes of dilution buffer are added to one volume of lysate.
(c) purifying a protein by chromatographic separation, wherein purification is performed by chromatographic separation by passing the diluted protein through one immobilized metal ion affinity *chromatography* (IMAC) column or one IMAC and one chitin column, and wherein the elution buffer for IMAC comprises 40 to 60 mM NaH₂PO₄, 270 to 330mM NaCl, 450 to 550mM imidazole, 9 to 11% glycerol at pH 7.5 to 8.5.

In preffered method of the invention for purifying the protein, the solubilization buffer comprises 2M urea and the buffer pH is about 12.0.

In preffered method of the invention for purifying the protein, the bacteria is an *E. coli* strain, even more preferably the bacteria is *E. coli* strain NiCo21(DE3) or NiCo23(DE3).

In preffered method of the invention for purifying the protein, the solubilization buffer comprises 40 to 60mM NaH₂PO₄, 270 to 330mM NaCl, 18 to 22mM imidazole, 9 to 11% glycerol, 1.8 to 2.2M urea at a pH in the range of from pH11.5 to 12.5; preferably wherein the solubilization buffer comprises 50mM NaH₂PO₄, 300mM NaCl, 20mM imidazole, 10% glycerol, 0.5mM PMSF, 5mM β-mercaptoethanol, 2M urea at pH12.0; preferably wherein the solubilization buffer comprises 50mM NaH₂PO₄, 300mM NaCl, 20mM imidazole, 10% glycerol, 0.5mM PMSF, 5mM β-mercaptoethanol, lysozyme (1mg/ml), 0.05% Triton™ X-100, benzonase (5U/ml), 2M urea at pH12.0.

In preffered method of the invention for purifying the protein, the dilution buffer comprises 40 to 60 mM NaH₂PO₄, 270 to 330mM NaCl, 18 to 22mM imidazole, 9 to 11 % glycerol at a pH in the range of from pH 7.5 to 8.5; preferably wherein the dilution buffer comprises 50mM NaH₂PO₄, 300mM NaCl, 20mM imidazole, 10% glycerol at pH 8.0.

In preffered method of the invention for purifying the protein, purification is performed by chromatographic separation by passing the protein through two columns wherein the protein is first passed through a chitin column and secondly through a nickel, cobalt or copper column; preferably wherein purification is performed by chromatographic separation by passing the protein through two connected chromatographic columns; preferably wherein the two connected columns are a chitin column and an IMAC nickel, cobalt or copper column.

In preffered method of the invention for purifying the protein, the elution buffer for IMAC comprises 50mM NaH₂PO₄, 300mM NaCl, 500mM imidazole, 10% glycerol at pH 8.0.

In preffered method of the invention for purifying the protein, the protein is a Exotoxin A fusion protein; preferably protein is a fusion protein comprising a targeting moiety, cell surface receptor ligand or signaling molecule; preferably wherein the protein is a fusion protein comprising an antibody or antigen-binding fragment thereof, wherein the protein is provided as a His-tagged SUMO protein; preferably wherein the protein is provided as a His-tagged SUMO protein and after purification by IMAC the His-tagged SUMO protein moiety is removed by incubation with a specific SUMO (ULP-1) protease.

In preffered method of the invention for purifying the protein, the method further comprises separating the protein by size-exchange chromatography (SEC); preferably wherein SEC is combined with buffer exchange; preferably wherein the buffer for buffer exchange is into PBS, optionally comprises glycerol; preferably wherein the buffer for buffer exchange is into PBS comprising 10% glycerol.

In preffered method of the invention for purifying the protein, the method further comprises concentrating the protein obtained by lyophilisation, spray drying, ultrafiltration or diafiltration.

In preffered method of the invention for purifying the protein, the Exotoxin A, Exotoxin A mutein or fragment thereof is an active Exotoxin A, active Exotoxin A mutein, active fragment of Exotoxin A or active fragment of an Exotoxin A mutein.

In preffered method of the invention for purifying the protein, the method further comprises testing the activity of the protein obtained; preferably comprises testing the cytotoxic activity of the protein obtained; preferably comprises testing the cytotoxic activity of the protein obtained by ADP-ribosylation assay and / or by neutral red uptake assay.

In preffered method of the invention for purifying the protein, the method further comprises conjugating the protein obtained with a targeting moiety, cell surface receptor ligand, signaling molecule or further cytotoxin.

In preffered method of the invention for purifying the protein, the method further comprises formulating the protein or conjugate obtained into a composition with one or more excipients; preferably further comprises formulating the protein or conjugate obtained into a pharmaceutical composition with one or more pharmaceutically-acceptable excipients.

It is also disclosed a method for purifying a protein comprising an Exotoxin A, Exotoxin A mutein or fragment thereof comprising:
(a) solubilizing a sample comprising the protein in a solubilization buffer containing urea and at alkaline pH,
(b) decreasing the concentration of urea and the pH by dilution using a dilution buffer, and / or by dialysis into a dilution buffer, prior to purification of the protein.

In preferred methods of the invention the solubilization buffer comprises a low concentration of urea. The combination of a low concentration of urea (e.g., in the range of from 1.8M to 2.2M, preferably 1.9M to 2.1M urea, more preferably about 2M (+/- 5%), most preferably 2M urea and high, alkaline pH (*e.g*., in the range of from pH11.5 to pH12.5, more preferably about pH12 (+/-5%), most preferably pH12) has never before been applied to purification of Exotoxin A proteins. This unique combination helps with protein recovery from both soluble fraction and IBs without their complete unfolding and thus allows for retention of the active form of Exotoxin A, muteins and fragments thereof. The method of invention is relatively fast, simple and buffer efficient comparing to traditional refolding protocols. The protein in buffer is stable enough to apply a simple dilution step to remove an excess of urea prior to purification. The method of the invention is reproducible as the purity and activity is comparable for different batches of isolated protein. Moreover, the purity and activity of protein obtained by the described method is higher than protein purified directly from soluble fraction.

In described method, the sample can be a partially purified sample of protein provided in the form of a lyophilized or dehydrated sample, solution, suspension or supernatant. The sample can be a wild type lyophilized Exotoxin A obtained by purification of Exotoxin A from *Pseudomonas aeruginosa.*

In a method of the invention, the protein can be a recombinant protein. When the protein is a recombinant protein, the sample can be a bacterial pellet obtained following expression of the recombinant protein in bacteria. As used herein, "recombinant" includes a protein produced using cells that do not have, in their native state, an endogenous copy of the DNA encoding the protein. The cells produce the recombinant protein because they have been genetically altered by the introduction of the appropriate isolated nucleic acid sequence. In methods of the invention the protein can be obtained by heterologous expression in *E*. *coli.* Any suitable *E*. *coli* strain can be used.

Preferably the bacteria is an *E*. *coli* strain, more preferably the bacteria is *E*. *coli* strain NiCo21(DE3) or NiCo23(DE3), most preferably the *E*. *coli* strain is NiCo21(DE3) from New England Biolabs. Due to specific modifications applied to homologic bacterial proteins of NiCo21(DE3) and NiCo23(DE3), the purified protein is obtained with very high purity.

The exotoxin A nucleic acid sequence can be codon-optimised for expression in *E.coli,* e.g., for expression of a recombinant Exotoxin A protein in *E.coli.*

In a method of the invention the solubilization buffer may contain urea at a concentration in the range of from 1.8 to 2.2M and have a buffer pH in the range of from 11.5 to 12.5, and solubilization buffer is a phosphate buffer. Preferably the solubilization buffer comprises 40 to 60mM NaH₂PO₄, 270 to 330mM NaCl, 18 to 22mM imidazole, 9 to 11% glycerol, 1.8 to 2.2M urea at a pH in the range of from pH11.5 to 12.5. More preferably the solubilization buffer comprises about 2M urea and the buffer pH is about 12.0. By "about" we mean plus or minus 5% of the stated value. Most preferably the solubilization buffer comprises 2M urea and the buffer pH is 12.0.

In an embodiment, solubilization buffer comprises 50mM NaH₂PO₄, 300mM NaCl, 20mM imidazole, 10% glycerol, 0.5mM PMSF, 5mM β-mercaptoethanol, 2M urea at pH12.0. This buffer is useful when the sample is a partially purified sample of protein.

In an embodiment the solubilization buffer comprises 50mM NaH₂PO₄, 300mM NaCl, 20mM imidazole, 10% glycerol, 0.5mM PMSF, 5mM β-mercaptoethanol, lysozyme (1mg/ml), 0.05% Triton™ X-100, benzonase (5U/ml), 2M urea at pH12.0. This buffer is useful when the sample is provided as a bacterial lysate or bacterial pellet.

According to methods of the invention the protein to be purified is provided as bacterial pellet. According to a preferred method of the invention the pellet is thoroughly resuspended in a solubilization buffer, suitably a phosphate buffer at as described herein, most preferably at pH12.0 and comprising 2M urea. The solution must be freshly prepared at room temperature (RT). The recommended concentration factor (ratio of the culture size to the amount of solubilization buffer) is in the range of from 30x to 40x. Preparation of the bacterial cell lysate from the resuspended pellet may be further assisted by mechanical cell disruption by sonication, passing the suspension through a cell disruptor or through the French press.

Preferably the dilution buffer comprises phosphate buffer without urea at a pH in the range of from pH 7.8 to 8.2. More preferably the dilution buffer comprises 40 to 60 mM NaH₂PO₄, 270 to 330mM NaCl, 18 to 22mM imidazole, 9 to 11 % glycerol at a pH in the range of from pH 7.5 to 8.5. Most preferably the dilution buffer comprises 50mM NaH₂PO₄, 300mM NaCl, 20mM imidazole, 10% glycerol at pH 8.0.

Dilution of the solution is a recommended method to decrease the urea concentration and pH. In a preferred method phosphate buffer without urea and at pH 8.0 ("lysis buffer") is added to freshly-prepared cell lysate. Preferably three volumes of dilution buffer are added to one volume of lysate. The solution is mixed gently and centrifuged at 4°C to remove cellular debris. Alternatively or in addition to dilution, dialysis can be used to decrease urea concentration and pH, but dialysis can be less favorable because it lengthens the purification procedure and may result in loss of protein. Undiluted bacterial lysate can be used for purification on IMAC chromatography, but this is not recommended because the efficiency of protein capture by nickel resin may be lower in the presence of either urea or elevated pH.

In the described method purification may be performed by chromatographic separation.

Purification may be performed by chromatographic separation by passing the protein through one or two immobilized metal ion affinity chromatography (IMAC) columns. Purification may be performed by chromatographic separation by passing the protein through two connected IMAC columns. The two connected IMAC columns may be a chitin column and a nickel, cobalt or copper column. In a preferred method, purification is performed so that the protein is first passed through a chitin column and secondly through a nickel, cobalt or copper column.

The elution buffer for IMAC preferably comprises 40 to 60 mM NaH₂PO₄, 270 to 330mM NaCl, 450 to 550mM imidazole, 9 to 11% glycerol at pH7.5 to 8.5. Most preferably the elution buffer for IMAC comprises 50mM NaH₂PO₄, 300mM NaCl, 500mM imidazole, 10% glycerol at pH 8.0.

It is also disclosed the method for solubilization and purification of recombinant Exotoxin A, recombinant Exotoxin A muteins and recombinant proteins comprising Exotoxin A fragment, comprising the steps of:
(a) providing an E. coli bacterial lysate containing overexpressed protein of interest,
(b) treating the bacterial lysate with a denaturing buffer containing 2M urea, wherein pH of a buffer is around 12.0,
(c) dilution of bacterial lysate obtained in (b) to decrease the urea concentration and to lower the pH to around 8.0,
(d) passing the lysate through IMAC chromatography column,
(e) separating the pure protein with SEC chromatography which is combined with buffer exchange.

In a variation, the invention provides a method for purification of recombinant Exotoxin A, recombinant Exotoxin A muteins and recombinant proteins containing Exotoxin A fragment comprising the alternative steps (a) to (d) of:
(a) providing E. coli bacterial lysate from strain NiCo21(DE3) or NiCo23(DE3) containing overexpressed protein of interest,
(b) treating the bacterial lysate with a denaturating buffer containing 2M urea, wherein pH of a buffer is around 12.0,
(c) dilution of bacterial lysate obtained in (b) to decrease the urea concentration and to lower the pH to around 8.0,
(d) passing the lysate through two connected IMAC chromatography columns (chitin column and nickel column).

Methods of the invention are advantageous when compared to other methods in the art, because of the use of a low concentration of urea (e.g., 2M) combined with elevated pH (e.g., 12.0). When the protein is expressed as a recombinant protein the whole bacterial pellet can be used for lysis and there is no need to separate pure IBs. Moreover, as the excess of urea can be removed by simple dilution, the purification steps can be reduced to use only two separate columns. Methods of the invention ensure high purity of the protein and batch-to-batch reproducible protein activity, which has been found to be higher than the activity of the protein obtained with the aid of traditional purification protocols.

In a method of the invention the protein can be a fusion protein. The protein can be a fusion protein comprising a targeting moiety, cell surface receptor ligand or signaling molecule. Preferably the protein is a fusion protein comprising an antibody or antigen-binding fragment thereof.

The term "targeting moiety" as used herein, refers to any molecule or agent that specifically recognizes and binds to a cell-surface marker, such that the targeting moiety can direct the delivery of the cytotoxin to a population of cells on which surface the receptor is expressed. Targeting moieties include, but are not limited to, antibodies (e.g., monoclonal antibodies), or fragments thereof, peptides, hormones, growth factors, cytokines, and any other natural or non-natural ligands such as, e.g., scaffold antigen binding proteins.

The fusion protein can be an immunotoxin, comprising a targeting moiety, such as an antibody or fragment thereof which retains antigen recognition capability and a cytotoxic moiety of cytotoxic Exotoxin A protein as described herein. The targeting moiety and cytotoxic moiety may be joined by a linker sequence. A "targeting moiety" is the portion of an immunoconjugate intended to target the immunoconjugate to a cell of interest. Typically, the targeting moiety is an antibody, a scFv, a dsFv, a Fab, or a F(ab')2.

The term "antibody," as used herein, refers to whole (also known as "intact") antibodies or antigen binding portions thereof that retain antigen recognition and binding capability. The antibody or antigen binding portions thereof can be a naturally-occurring antibody or antigen binding portion thereof. The antibody or antigen binding portion thereof can be in monomeric or polymeric form. Also, the antibody or antigen binding portion thereof can have any level of affinity or avidity for the cell surface marker. Desirably, the antibody or antigen binding portion thereof is specific for the cell surface marker, such that there is minimal cross-reactivity with other peptides or proteins.

The antibody may be monoclonal or polyclonal and of any isotype, e.g., IgM, IgG (e.g. IgG, IgG2, IgG3 or IgG4), IgD, IgA or IgE. Complementarity determining regions (CDRs) of an antibody or single chain variable fragments (Fvs) of an antibody against a target cell surface marker can be grafted or engineered into an antibody of choice to confer specificity for the target cell surface marker upon that antibody. For example, the CDRs of an antibody against a target cell surface marker can be grafted onto a human antibody framework of a known three dimensional structure to form an antibody that may raise little or no immunogenic response when administered to a human. In a preferred embodiment, the targeting moiety is a monoclonal antibody or an antigen binding portion of the monoclonal antibody, more preferably a human monoclonal antibody or an antigen binding portion of the human monoclonal antibody.

The antigen-binding portion can be any portion that has at least one antigen binding site, such as, e.g., the variable regions or CDRs of the intact antibody. Examples of antigen binding portions of antibodies include, but are not limited to, a heavy chain, a light chain, a variable or constant region of a heavy or light chain, a single chain variable fragment (scFv), or an Fc, Fab, Fab', Fv, or F(ab)2' fragment; single domain antibodies (such as VHH or VH), helix-stabilized antibodies; triabodies; diabodies; single-chain antibody molecules ("scFvs"); disulfide stabilized antibodies ("dsFvs"), and domain antibodies ("dAbs").

In a preferred embodiment the antibody is a single-chain variable fragment (scFv), which is a fusion protein of the variable regions of the heavy (VH) and light chains (VL) of immunoglobulins, connected with a short linker peptide usually of ten to about 25 amino acids. The linker is usually glycine-rich to confer flexibility, and may contain serine or threonine for solubility. The linker can either connect the N-terminus of the VH with the C-terminus of the VL, or vice versa. ScFv retain the specificity of the original immunoglobulin, despite absence of the constant regions and the introduction of the linker.

A wide variety of metal affinity ion chromatography (IMAC) resins can be used in the methods of invention, e.g. nickel, cobalt or copper. Chitin resin can be used for purification of bacterial lysate obtained from E. coli strain NiCo21(DE3). Chitin resin can be used to remove CBD-tagged endogenous *E. coli* metal binding proteins.

Depending on the method of protein production and further protein application, the removal of histidine tag might be required. In preferred embodiments, a His-tag is fused to the N-terminus of a small ubiquitin-related modifier (SUMO) that is directly fused to N-termini of protein of interest. The SUMO protein with His-tag can be cleaved off by addition of specific SUMO (ULP-1) protease.

The protein can be provided as a His-tagged SUMO protein. When the protein is provided as a His-tagged SUMO protein, after purification by IMAC the His-tagged SUMO protein moiety may be removed by incubation with a specific SUMO (ULP-1) protease.

A method according to the invention may further comprise separating the protein by size-exclusion chromatography (SEC). SEC may be combined with buffer exchange. The buffer for buffer exchange may be PBS, optionally comprising glycerol. Preferably the buffer for buffer exchange is into PBS comprising 10% glycerol.

A final protein purification step can be performed by size exclusion chromatography (SEC). Depending on the size of a protein of interest, different SEC resins can be used. Advantageously, the inventors found that size exclusion chromatography step is suitable for simultaneous protein separation and buffer exchange. SEC can be performed in PBS with addition of 10% glycerol to improve protein stability. The combined protein separation with buffer exchange eliminates the additional step, *i.e.,* dialysis or using chromatography on desalting columns. This prevents against loss of yield and shortens the process of protein purification.

A method according to the invention may further comprise concentrating the protein obtained by lyophilisation, spray drying, ultrafiltration or diafiltration.

The described methods are generally applicable to proteins comprising Exotoxin A, Exotoxin A muteins or Exotoxin A fragments produced as soluble proteins and as aggregates in inclusion bodies.

The Exotoxin A, Exotoxin A mutein or fragment thereof may be an active Exotoxin A, active Exotoxin A mutein, active fragment of Exotoxin A or active fragment of an Exotoxin A mutein.

The described methods are also useful for purification of inactive Exotoxin A muteins and fragments thereof, such as the triple alanine mutant Q483A, D484A, D488A.

*Pseudomonas* exotoxin A (PE) is a bacterial toxin (molecular weight 66 kD) secreted by *Pseudomonas aeruginosa.* Immediately after translation the precursor Exotoxin A protein consists of 638 amino acids (http://www.uniprot.org/uniprot/P11439, SEQ ID NO: 1); post-translational processing of the 638 amino acid protein results in Exotoxin A protein of 613 amino acids (SEQ ID NO: 2). The native, wild-type PE sequence is disclosed in U.S. Pat. No. 5,602,095. Native, wild-type PE includes three structural domains that contribute to cytotoxicity. Domain la (amino acids 1-252) mediates cell binding, domain II (amino acids 253-364) mediates translocation into the cytosol, and domain III (amino acids 400-613) mediates ADP ribosylation of elongation factor 2. While the structural boundary of domain III of PE is considered to start at residue 400, it is contemplated that domain III may require a segment of domain Ib to retain ADP-ribosylating activity. Accordingly, functional domain III is defined generally as residues 395-613 of Exotoxin A (SEQ ID NO: 3), this is also termed the "minimum cytotoxic fragment". The function of domain Ib (amino acids 365-399) remains undefined. Without wishing to be bound by theory, it is believed that the cytotoxic activity of Exotoxin A occurs through the inhibition of protein synthesis in eukaryotic cells, e.g., by the inactivation of elongation factor 2 (EF-2) by ADP-ribosylation.

Exotoxin A proteins, that is, Exotoxin A, muteins of Exotoxin A and proteins comprising fragments of Exotoxin A or fragments of Exotoxin A muteins can be modified in any number of ways, such that the therapeutic or prophylactic efficacy is increased through the modification. For instance, Exotoxin A, muteins or fragments thereof can be conjugated or fused either directly or indirectly through a linker to a targeting moiety to provide a chimeric or fusion molecule comprising (a) a targeting moiety conjugated or fused to (b) any of the Exotoxin A proteins described herein. The practice of conjugating compounds to targeting moieties is known in the art.

In preferred embodiments, the protein to be purified comprises an active Exotoxin A protein that retains a biological activity of Exotoxin A, e.g., ADP-ribosylation activity and / or cytotoxicity, for example cytotoxicity assessed by a neutral red uptake assay. Active Exotoxin A proteins include unmodified three-domain Exotoxin A, muteins of Exotoxin A, active fragments of Exotoxin A, active fragments of Exotoxin A muteins, active fragments of Exotoxin A that comprise the minimum cytotoxic fragment and active fragments of muteins of Exotoxin A that comprise the minimum cytotoxic fragment.

Exotoxin A muteins are modified forms of exotoxin A that have been modified by addition, deletion or substitution of one or more amino acids and which retain a biological activity of Exotoxin A, e.g., ADP-ribosylation activity and / or cytotoxicity, for example cytotoxicity assessed by a neutral red uptake assay.

Muteins can be produced by single or multiple insertions, deletions or substitutions of amino acids. Muteins can also be produced by addition of different motifs, e.g.: i) targeting motifs (*e.g*., nuclear localisation signals), ii) motifs recognized by different cellular proteases, iii) motifs resembling native structure of the toxin (*e.g*., substitution of whole translocation domain of the toxin).

Exotoxin A fragments are fragments of a wild type Exotoxin A or mutein Exotoxin A that retain a biological activity of Exotoxin A, *e.g*. ADP-ribosylation activity and / or cytotoxicity, for example cytotoxicity assessed by a neutral red uptake assay.

Described methods are also useful for purification of inactive Exotoxin A muteins and fragments thereof, such as the triple alanine mutant Q483A, D484A, D488A. The mutations, proposed by Susan P. Yates and A. Rod Merill [Yates, S. P. and A. R. Merrill (2001). A catalytic loop within Pseudomonas aeruginosa exotoxin A modulates its transferase activity. J Biol Chem 276(37): 35029-35036] cause almost 100% loss of protein ADP-rybosylation activity without interfering with its substrate binding capacity.

A method according to the invention may further comprise testing the activity of the protein obtained. In particular, testing the cytotoxic activity of the protein obtained. The activity of the protein obtained may be tested by ADP-ribosylation assay and / or by neutral red uptake assay. The cytotoxicity of the protein obtained may be tested by neutral red uptake assay.

A method according to the invention may further comprise conjugating the protein obtained with a targeting moiety, cell surface receptor ligand, signaling molecule or further cytotoxin. Suitable methods for conjugation of proteins to form conjugates are known in the art.

A method according to the invention may further comprise formulating the protein or conjugate obtained into a composition with one or more excipients. The purified protein or conjugate composition essentially does not comprise urea. When purified, the protein or conjugate composition can be stored at -80°C for long term storage.

Proteins purified by a method of the invention and conjugates thereof are suitable for both laboratory and oncology research. Unmodified Exotoxin A and muteins of Exotoxin A can be used to study toxin mode of action, cellular trafficking and cytotoxic response of different cell lines. Unmodified Exotoxin A and muteins of Exotoxin A can be chemically conjugated to other proteins or expressed as fusions with other proteins, e.g., antibodies, to create immunotoxins. Unmodified Exotoxin A and muteins of Exotoxin A can be chemically conjugated to polymer or protein nanoparticles. Immunotoxins and conjugated proteins can be used in cancer treatment.

A method according to the invention may further comprise formulating the protein or conjugate obtained into a pharmaceutical composition with one or more pharmaceutically-acceptable excipients.

It is also described a protein, conjugate or composition obtained by a method of the invention for use as a medicament.

It is also described a protein, conjugate or composition obtained by a method of the invention for use in the manufacture of a medicament for the treatment of the human or animal body.

It is also described a method of treatment of the human or animal body comprising administration of a recombinant protein, conjugate or composition obtained by a method of the invention.

It is also described a protein, conjugate or composition obtained by a method of the invention for use in treatment of cancer.

It is also described a protein, conjugate or composition obtained by a method of the invention for use in the manufacture of a medicament for the treatment of a cancer of the human or animal body.

It is also described a method of treatment of a cancer of the human or animal body comprising administration of a protein or composition obtained by a method of the invention.

### Description of the drawings

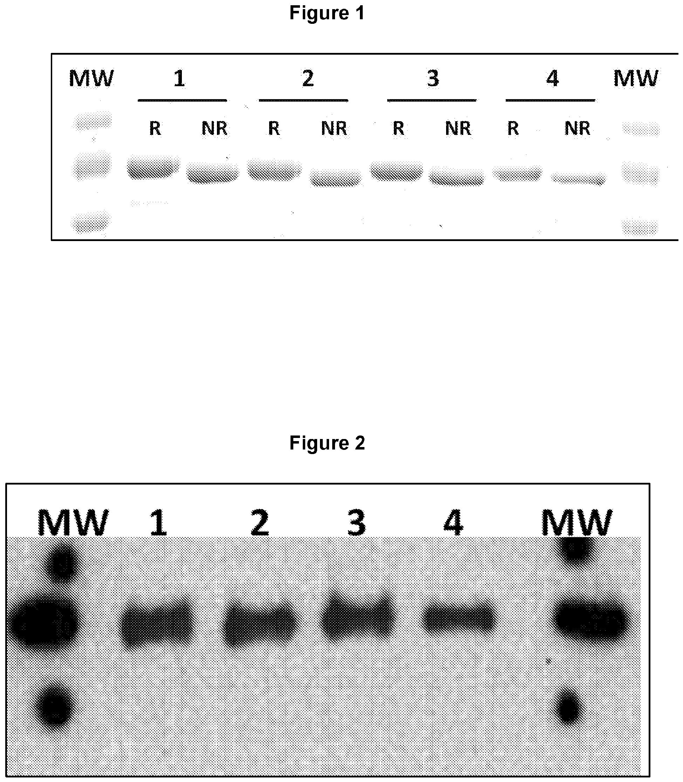

**Figure 1****.** The SDS-PAGE analysis of purified proteins in reducing (R) and non-reducing (NR) conditions. Lanes 1-4: (1) commercially-available Exotoxin A prepared according to manufacturer's protocol; (2) commercially-available Exotoxin A prepared according to method described in Example 3, (3) Exotoxin A purified according to method of invention described in Example 2, (4) Exotoxin A purified from soluble fraction described in Example 1, respectively. Lane MW is a molecular weight marker (100, 70 and 55kDa).
**Figure 2****.** The Western blot analysis of proteins. Proteins were separated under reducing conditions and detected with anti-Exotoxin A antibody produced in rabbit (Sigma Aldrich). Lanes 1-4: (1) commercial Exotoxin A, (2) commercial Exotoxin A prepared according to method of invention, (3) Exotoxin A purified according to method of invention, (4) Exotoxin A purified from soluble fraction, respectively. Lane MW is a molecular weight marker (100, 70 and 55kDa).

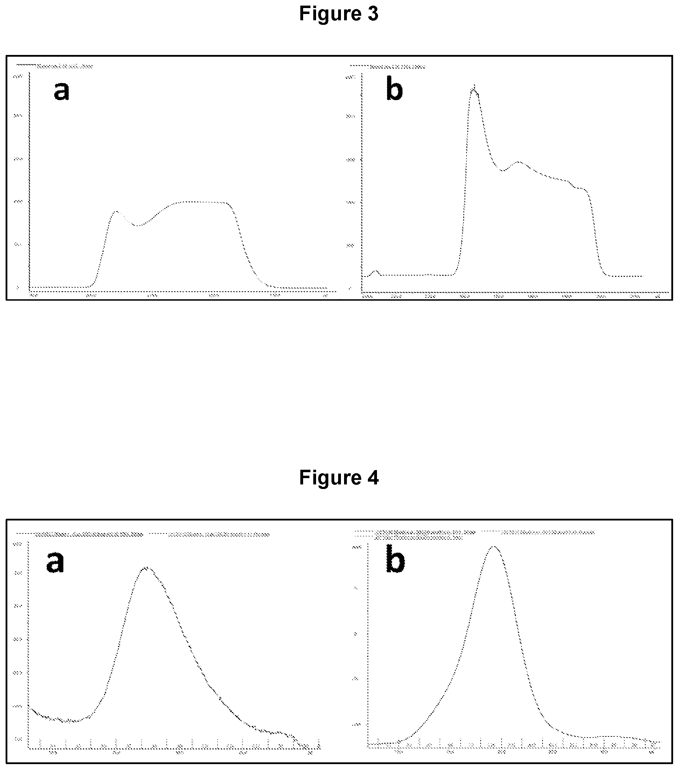

**Figure 3****.** Chromatograms of Exotoxin A purification on NiNTA column; a) purification from soluble fraction as described in Example 1, b) purification of Exotoxin A using method of inventionas described in Example 2. Chromatography was performed on Äkta Purifier 10 FPLC System.
**Figure 4****.** Chromatograms of Exotoxin A purification on size exclusion column (SEC); a) purification from soluble fraction as described in Example 1, b) purification of Exotoxin A using method of invention as described in Example 2. Chromatography was performed on Äkta Purifier 10 FPLC System.

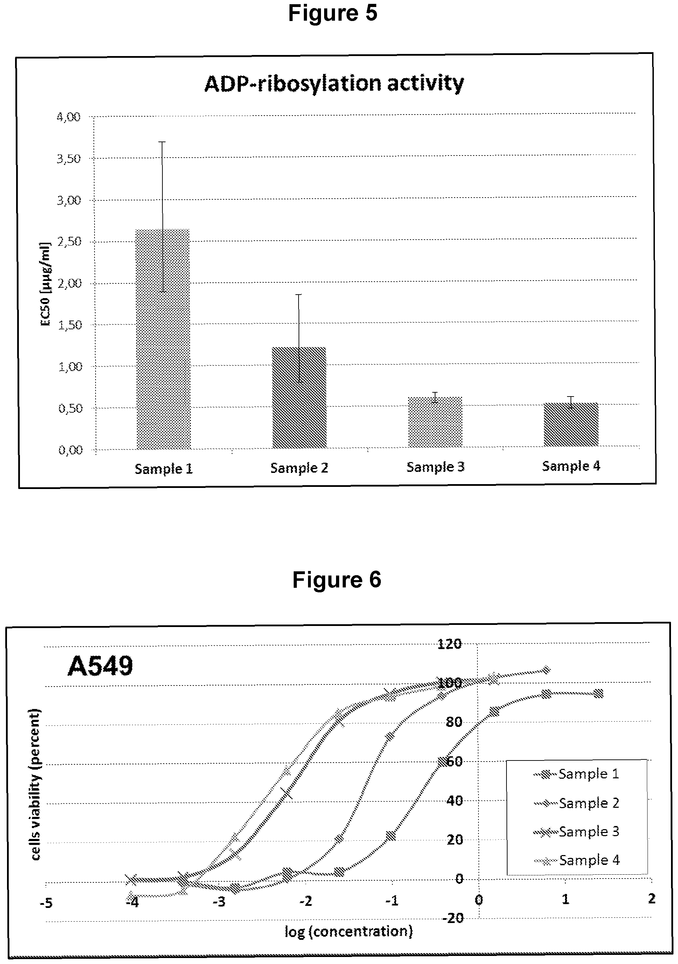

**Figure 5****.** Analysis of *in vitro* ADP-ribosylation activity of Exotoxins A. Samples 1-4: (1) commercial Exotoxin A, (2) commercial Exotoxin A prepared according to method of invention, (3) Exotoxin A purified from soluble fraction, (4) Exotoxin A purified according to method of invention, respectively.
**Figure 6****.** Cytotoxicity measurement of Exotoxin A on A549 cell line. The x-axis shows the log10 concentration of protein, the y-axis shows % cell viability. Samples 1-4: commercial Exotoxin A (Example 3a,■), commercial Exotoxin A prepared according to method of invention (Example 3b,◆), Exotoxin A purified from soluble fraction (Example 1, X), Exotoxin A purified according to method of invention (Example 2, ▲), respectively

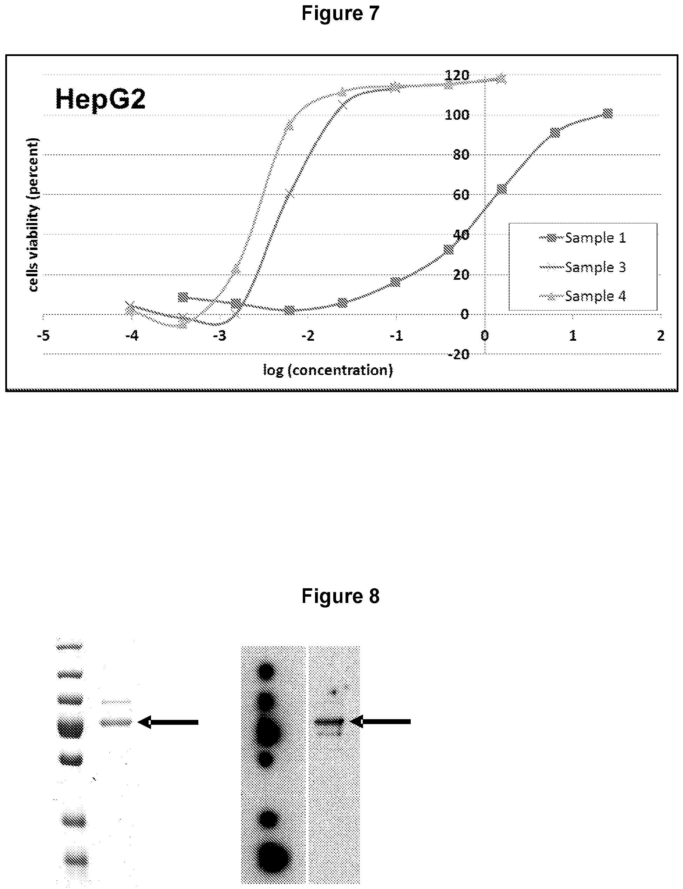

**Figure 7****.** Cytotoxicity measurement of Exotoxins A on HepG2 cell line. Samples 1, 3, 4: commercial Exotoxin A (Example 3a, ■), Exotoxin A purified from soluble fraction (Example 1, X), Exotoxin A purified according to method of invention, respectively(Example 2, ▲).
**Figure 8****.** SDS-PAGE in reducing conditions analysis and Western blot for PEantiC immunotoxin purified by a method of the invention, the purity was 81.8%. Figure 8 shows the results of SDS-PAGE analysis (left panel) and Western blot (right panel), the bands corresponding to purified immunotoxin PEantiC are indicated by arrows.

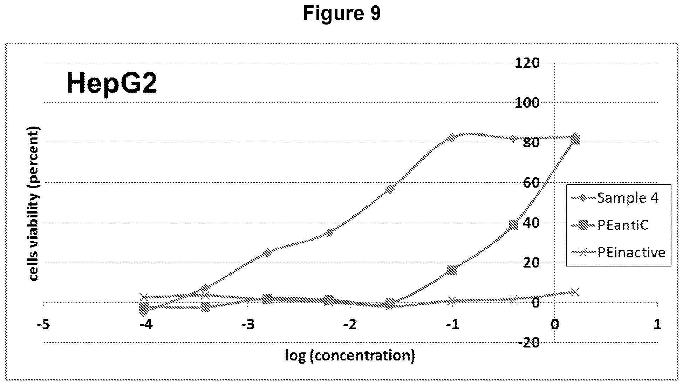

**Figure 9****.** Cytotoxicity measured on A549 cell line as described in Example 6. The x-axis shows the log10 concentration of protein, the y-axis shows % cell viability for: immunotoxin PEantiC (Example 6, ■), PEinactive, an inactive triple-alanine Exotoxin A Q483A, D484A, D488A mutein (X), and unmodified Exotoxin A (Example 2,◆). All proteins were purified according to method of invention.

The purification method of the invention is described in detail with the aid of the following examples. Various modifications that may be apparent to one in the art are intended to fall within the scope of this invention.

### Examples

### Example 1: Expression and purification of recombinant Exotoxin A from soluble fraction (prior art method)

### 1. Cloning and expression of Exotoxin A

A synthetic gene encoding Exotoxin A was codon-optimized for expression in *E. coli* (SEQ ID NO: 5). Sequence and ligase-independent cloning was used to clone the gene an into expression vector based on pET28a from Novagene to create pET28SUMO (SEQ ID NO: 6), in which the Exotoxin A protein (SEQ ID NO: 4) was fused to a His-tagged SUMO protein. A small-ubiquitin-related modifier (SUMO) is reported to improve protein refolding and solubilization and can be cleaved from the protein using a highly specific ULP-1 protease (SUMO protease). The vector was sequenced to confirm that the cloning was successful and correct.

The pETSUMO vector carrying the codon-optimized Exotoxin A coding sequence (SEQ ID NO: 5) was transformed into chemocompetent *E. coli* strain NiCo21(DE3) by heat shock. The transformation mixture was plated out on agar plates supplemented with 1% glucose and kanamycin (50µg/ml). Bacteria were grown on LB agar plates for 24h at 37°C. The preculture was inoculated with single colony and grown in TB medium for 16h at 37°C. Fresh TB medium was warmed to 37°C and inoculated with seed culture at a ratio of 1:100 (culture:media). The culture was induced with 0.5mM IPTG when OD reached 0.4 and further grown for 14h at 23°C. Bacteria were then collected by centrifugation to form a pellet.

### 2. Bacterial pellet lysis

The bacterial pellet was suspended in lysis buffer, at a 1:4 weight:volume ratio, the lysis buffer containing 50mM NaH₂PO₄, 300mM NaCl, 20mM imidazole, 10% glycerol, 0.5mM PMSF, 5mM β-mercaptoethanol, lysozyme (1mg/ml), 0.05% Triton™ X-100, benzonase (5U/ml), pH 8.0. Resuspended lysate was sonicated and centrifuged. The supernatant was collected and immediately processed. The lysate and supernatant were kept at 4°C during all procedures.

### 3. Protein purification on double IMAC chromatography

Supernatant containing the protein of interest was applied on two connected chromatography columns, i.e., a first column containing chitin resin (NEB) and a subsequent column filled with NiNTA Superflow resin (Qiagen). Each of the columns was equilibrated using the same lysis buffer (50mM NaH₂PO₄, 300mM NaCl, 20mM imidazole, 10% glycerol, pH 8.0). The supernatant was loaded with a constant flow rate of 0.1 ml/min. Columns were washed using 8 column volumes (8CV) of buffer and were disconnected afterwards. The single NiNTA column was then washed with buffer containing high salt concentration to remove any material that had non-specifically bound (50mM NaH₂PO₄, 2000mM NaCl, 20mM imidazole, 10% glycerol, pH 8.0). The protein was eluted in gradient elution mode with buffer 50mM NaH₂PO₄, 300mM NaCl, 500mM imidazole, 10% glycerol, pH 8.0. Collected fractions were pooled together and SUMO protease was added to remove the SUMO-tag.

### 4. Protein purification on SEC chromatography

After SUMO-tag removal the protein was filtered and loaded on size exclusion column (HiLoad Superdex 200, GE Healthcare) equilibrated with phosphate-buffered saline, to which 10% glycerol, pH 7.4 was added. The fractions containing the Exotoxin A protein of interest (SEQ ID NO: 4) were pooled and concentrated on Vivaspin Turbo. Protein concentration was measured by Bradford protein assay, analyzed on SDS-PAGE (Figure 1, lane 4) and confirmed by Western blot analysis (Figure 2, lane 4) against anti-Pseudomonas Exotoxin A (Sigma Aldrich). Protein purity was assessed by densitometry (Table 1).

The purity and activity of the protein are presented in Table.1

### Example 2: Expression and purification of recombinant Exotoxin A using a method of the invention

### 1. Cloning and expression of Exotoxin A

Cloning and expression was performed as described in Example 1.

### 2. Bacterial pellet lysis

The bacterial pellet was suspended in solubilization buffer in 1:10 weight:volume ratio, containing: 50mM NaH₂PO₄, 300mM NaCl, 20mM imidazole, 10% glycerol, 0.5mM PMSF, 5mM β-mercaptoethanol, lysozyme (1mg/ml), 0,05% Triton™ X-100, benzonase (5U/ml), 2M urea, pH 12.0. Resuspended lysate was sonicated and slowly diluted 4x with buffer devoid of urea and at pH 8.0 (50mM NaH₂PO₄, 300mM NaCl, 20mM imidazole, 10% glycerol, pH 8.0). The solution was gently mixed and centrifuged. The supernatant was collected and immediately processed. The lysate was kept in room temperature while centrifuged supernatant was kept in 4°C.

### 3. Protein purification on double IMAC

Supernatant containing the protein of interest was applied on two connected chromatography columns, i.e. column containing chitin resin (NEB) and subsequent column filled with NiNTA Superflow resin (Qiagen). Both columns were equilibrated with the same lysis buffer (50mM NaH₂PO₄, 300mM NaCl, 20mM imidazole, 10% glycerol, pH 8.0). The supernatant was loaded with the constant flow rate of 0.1 ml/min. Columns were washed with 8 column volumes of buffer and disconnected afterwards. The single NiNTA column was then washed with buffer containing high salt concentration to remove any non-specific binding material (50mM NaH₂PO₄, 2000mM NaCl, 20mM imidazole, 10% glycerol, pH 8.0). The protein was eluted in gradient elution mode with buffer 50mM NaH₂PO₄, 300mM NaCl, 500mM imidazole, 10% glycerol, pH 8.0. Collected fractions were pooled together and SUMO protease was added to remove the SUMO-tag.

### 4. Protein purification on SEC

After SUMO-tag removal the protein was filtered and loaded on a size exclusion column (HiLoad Superdex 200, GE Healthcare) equilibrated with phosphate-buffered saline containing 10% glycerol, pH 7.4. The fractions containing protein of interest were pooled and concentrated using a Vivaspin Turbo. Protein concentration was measured by Bradford protein assay, analyzed on SDS-PAGE (Figure 1, lane 3) and confirmed by Western blot analysis against anti-Pseudomonas Exotoxin A (Sigma Aldrich) (Figure 2, lane 3). Protein purity was assessed by densitometry.

The purity and activity of the protein are presented in Table.1

### Example 3: Reconstitution of lyophilized Exotoxin A using a mild solubilization method

### Example 3a

Lyophilized Exotoxin A from *Pseudomonas aeruginosa* was obtained from Sigma Aldrich.

A first sample was prepared according to the manufacturer's recommendations, *i.e.*, reconstituted in 1ml of ultrapure water resulting in soluble protein with concentration of 0.5 mg/ml in buffer 10mM Tris-HCI, pH 8.0.

### Example 3b

Protein processing according to a method of invention.

Lyophilized Exotoxin A from *Pseudomonas aeruginosa* was obtained from Sigma Aldrich.

Lyophilized protein was reconstituted in 4ml buffer containing 50mM NaH₂PO₄, 300mM NaCl, 20mM imidazole, 10% glycerol, 0.5mM PMSF, 5mM β-mercaptoethanol 2M urea, pH 12.0. The solution was incubated at room temperature for 1h. The sample was 4x diluted with lysis buffer devoid of urea and at pH 8.0 (50mM NaH₂PO₄, 300mM NaCl, 20mM imidazole, 10% glycerol, pH8.0) To mimic the IMAC step the whole sample was applied to a desalting column to exchange the buffer. The toxin was kept overnight in 4°C in buffer containing 50mM NaH₂PO₄, 300mM NaCl, 250mM imidazole, 10% glycerol, pH8.0. The sample was loaded on size exclusion column (HiLoad Superdex 200, GE Healthcare) equilibrated with phosphate buffered saline containing 10% glycerol, pH 7.4. The fractions containing protein of interest were collected and concentrated on Vivaspin Turbo. Protein concentration was measured by Bradford protein assay.

The purity and activity of the protein are presented in Table.1

**Table 1. Comparison of observed purity, ADP-rybosylation activity and cytotoxicity of toxins obtained from soluble fraction and toxins purified according to a method of invention (2, 3b) or by the manufacturer's protocol (3a).**

| **Toxin** | **Protocol** | **Protein purity** | **ADP-ribosylation activity EC₅₀ [µg/µl]** | **Cytotoxicity IC₅₀ [ng/µl]** | |
|---|---|---|---|---|---|
| | | | | **A549** | **HepG2** |
| Example 1 | Soluble fraction | 63.0% | 0.60 | 7.5 | 5.3 |
| Example 2 | Method of the invention | 95.1% | 0.52 | 4.8 | 2.6 |
| Example 3a | Manufacturer's Protocol | 100% | 2.65 | 269.6 | 869.1 |
| Example 3b | Method of the invention | 100% | 1.22 | 52.9 | not tested |

### Example 4: Determination of the ADP-rybosylation activity of the purified proteins

Toxins purified as described in Example 1, Example 2, Example 3a and Example 3b were tested to determine their ADP-ribosylation activity *in vitro.*

### 1. Purification of eukaryotic Elongation Factor 2 (eEF2)

His-tagged eEF2 was expressed in *S. cerevisiae* in 12L of yeast extract peptone dextrose medium at 30°C for 22h. Pellets obtained after centrifugation were frozen in liquid nitrogen and rapidly disrupted in a stainless-steel mill. The disrupted cells were suspended in 1000 ml of lysis buffer (50mM NaH₂PO₄, 300mM NaCl, 20mM imidazole, 10% glycerol, pH 8.0). The lysate was sonicated, centrifuged and filtered. The lysate was applied to a NiNTA (Qiagen) column. The column was washed with buffer containing a high salt concentration (50mM NaH₂PO₄, 2000mM NaCl, 20mM imidazole, 10% glycerol, pH 8.0). Protein was eluted from column in gradient elution mode (50mM NaH₂PO₄, 300mM NaCl, 500mM imidazole, 10% glycerol, pH 8.0). The eluate was applied to a size exclusion column (HiLoad Superdex 200, GE Healthcare). The column was equilibrated with phosphate buffered saline (PBS) containing 10% glycerol, pH 7.4. Pure eEF2 was collected and concentrated using a Vivaspin Turbo. Protein concentration was determined by Bradford method (Bradford Protein Assay, BioRad).

Protein identity was confirmed by Western blot analysis against anti-EF2 antibody (Sigma Aldrich).

### 2. ADP-ribosylation activity assay

U16 MaxiSorp plates (Nunc) were precoated with goat anti-mouse antibody (GAM) (Dako). Antibody was diluted 1:100 in PBS and 100µl of the mixture was placed in each well for 2h. To avoid cross-contamination every second column of wells was coated. Wells were washed three times with fresh PBST (PBS with 0.05% Tween® 20), soaked for 5 minutes and washed three times again. GAM pre-coated wells were incubated overnight with 100µl of PBS with penta-his antibody (Qiagen) (to a final concentration of antibody at 5µg/ml). The next day wells were washed with PBST.

100µl of reaction mix contained 1.2µg of His-tagged eEF2, 2µl of biotinylated NAD+ (250 µM), 10 µl BSA (3mg/ml), 10µl L-arginine (100mM), 0.5mM NaCl, 1µl of toxin (at concentrations ranging from 100µg/ml to 0.1 µg/ml), 50mM TrisHCl, 1mM EDTA, 1mM DTT, pH 7.6. The reactions were incubated for 2h at 37°C and transferred to freshly-washed, coated wells. Each toxin was tested in triplicate on a separate plate with inactivated Exotoxin A as a background indicator on each plate.

The inactivated Exotoxin A used was the three alanine mutant (Q483A, D484A, D488A). The protein was purified according to a method of the invention as described in the examples.

The reaction mixtures were incubated on a 96-well plate for 2h at 21°C without shaking. Wells were washed with PBST and incubated with blocking solution (50 mg/ml dry milk powder in 200 µl PBS) for 30 min at 21°C followed by 30 min at 37°C. Wells were washed with PBST and incubated with streptavidin-HRP conjugate (Sigma Aldrich) for 30 min at 21°C (1 µg/ml of antibody in 100µl buffer containing 10µl FBS and 3mg BSA in PBS). Wells were washed with PBST and additionally incubated with PBST for 30min and washed again. 100µl of 3,3',5,5'-tetramethylobenzidine (0.1 mg/ml in 0.01% H₂O₂, 0.05M citric-phosphate buffer, pH 5.0) was added to each well for color development. To stop the reaction after 30 min, 50µl of 2M H₂SO₄ was added. The absorbance in each well was measured at 450nm and 490nm, using an Enspire microplate reader (Perkin Elmer).

The summary of protein activity is presented in Table 1 above.

Unexpectedly proteins obtained by the method for purifying the protein comprising an Exotoxin A, Exotoxin A mutein or fragment thereof according to the invention have higher ADP-rybosylation activity which might be the result of more relaxed conformation and/or bigger distance between catalytic amino acid residues caused by temporary contact with urea and/or alkaline pH.

### Example 5: Determination of the cytotoxic activity of the purified proteins

### 1. Cell culture

The HepG2 cells (*hepatocellular carcinoma* cell line) and A549 cells (*human lung carcinoma* cell line) were obtained from the ATCC (Manassas, USA) and were maintained under standard conditions in Dulbecco's Modified Eagle's Medium supplemented with 10% fetal bovine serum at 37°C in a humidified atmosphere of 5% CO₂. The passage number range for both cell lines was maintained between 20 and 25.

### 2. Cytotoxicity measurement

To estimate the cytotoxicity of the toxins investigated, the neutral red uptake assay was used [Repetto, G., del Peso, A., Zurita, J.L. (2008) Neutral red uptake assay for the estimation of cell viability/cytotoxicity. Nat Protoc 3, 1125-1131]. HepG2 and A549 cell lines were seeded into 96-well plates at a density of 1.5 × 10⁴ cells per well, and after 24 h cells were treated with increasing concentrations of the investigated toxins for an additional period of time. After that time, incubation medium was removed, and cells were washed with cold PBS. After washing, cells were incubated with 50µg/ml neutral red in HBSS for 3h. Following incubation, the neutral red solution was removed, cells were washed with PBS, cell-bound dye was extracted using a solution containing 50% ethanol and 1% acetic acid by gentle shaking for 10 min, and a 550-nm absorbance was determined using a Sunrise microplate reader (Tecan, Männedorf, Switzerland). The IC₅₀ values were calculated from linear dose-response curves using GraphPad Prism 5 software.

### Example 6: Expression and purification of an Exotoxin A immunotoxin: PEantiC

### 1. Cloning and Expression of PEantiC immunotoxin

The PEantiC vector PEantiCP28S (SEQ ID NO: 11) was generated for the expression of PEantiC. The PEantiC immunotoxin (SEQ ID NOS: 7 and 16) has an anti-CEA scFv (SEQ ID NOS: 8 and 13) joined by a linker (SEQ ID NOS: 9 and 14) to an exotoxin fragment that contains domains II, IB and III (SEQ ID NOS: 10 and 15). The PEantiC sequence was provided with an N-terminal SUMO tag (encoded by SEQ ID NO: 12). The PEantiCP28S vector was transformed into *E.coli* NiCo21(DE3) and the PEantiC was expressed as described in Example 1.

### 2. Bacterial pellet lysis

Bacterial pellet lysis was performed as described in Example 2.

### 3. Protein purification on double IMAC and SEC

Protein purification on double IMAC and SEC was performed as described in Example 2. Figure 8 shows the results of SDS-PAGE analysis (left panel) and Western blot (right panel), the bands corresponding to purified immunotoxin PEantiC are indicated by arrows.

### 4. Determination of the cytotoxic activity of the purified proteins

Cytotoxicity was assessed using A549 cells as described in Example 5. Figure 9 shows the cytotoxic activity of immunotoxin PEantiC (■), PEinactive, an inactive triple-alanine Exotoxin A Q483A, D484A, D488A mutein (X), and unmodified Exotoxin A (◆).

### Sequence Listing Information

**SEQ ID NO: 1** shows the wild type sequence of the *Pseudomonas aeruginosa* precursor Exotoxin A protein (638 amino acids) (source: http://www.uniprot.org/uniprot/P11439), amino acids 1 to 15 are the signal peptide, Domain IA, amino acids 26 to 277 (252 amino acids in length) is required for target cell recognition; Domain II, amino acids 278 to 389 (112 amino acids in length) is required for translocation in target cell cytoplasm; Domain IB is amino acids 390 to 429 (40 amino acids in length); Domain III, amino acids 430 to 638 (209 amino acids in length) is required for ADP-ribosylation activity
**SEQ ID NO: 2** shows the wild type (native) sequence of functional Exotoxin A (613 amino acids) Domain IA (amino acids 1-252) mediates cell binding, domain II (amino acids 253-364) mediates translocation into the cytosol, and domain III (amino acids 400-613) mediates ADP ribosylation of elongation factor 2.
**SEQ ID NO: 3** shows the "minimum" cytotoxic fragment of ExoA. Although the structural boundary of domain III of PE is considered to start at residue 400, it is believed that domain III may require a short segment of domain IB to retain ADP-ribosylating activity. Accordingly, functional cytotoxic domain III is defined generally as residues 395-613 of Exotoxin A.
**SEQ ID NO: 4** shows the amino acid sequence of the Exotoxin A protein (613 amino acids) used in the examples, which differs from SEQ ID NO: 2 by one amino acid, due to introduction of a restriction site in the coding DNA, this amino acid change does not affect the functional properties of the Exotoxin A protein.
**SEQ ID NO: 5** shows the DNA sequence encoding the Exotoxin A that was used in the examples, which was codon-optimized for expression in *E.coli.*
**SEQ ID NO: 6** shows the DNA sequence of the pET28SUMO plasmid containing the DNA sequence encoding the codon-optimized Exotoxin A (SEQ ID NO: 5) used in examples 1, 2.
**SEQ ID NO: 7** shows the amino acid sequence of the PEantiC immunotoxin: anti-CEA scFv linked via a linker to a fragment of Exotoxin A (Domains II, IB and III) as encoded by the PEantiC vector construct, PEantiCP28S.
**SEQ ID NO: 8** shows the amino acid sequence of the anti-CEA scFv as encoded by the PEantiC construct PEantiCP28S.
**SEQ ID NO: 9** shows the amino acid sequence of the linker between the anti-CEA scFv and fragment of Exotoxin A (Domains II, IB and III) as encoded by the PEantiC construct PEantiCP28S.
**SEQ ID NO: 10** shows the amino acid sequence of the fragment of Exotoxin A (Domains II, IB and III) as encoded by the PEantiC construct: PEantiCP28S.
**SEQ ID NO: 11** shows the DNA sequence of the PEantiC construct (PEantiCP28S).
**SEQ ID NO: 12** shows the DNA sequence of the Sumo-tag in the PEantiC construct PEantiCP28S.
**SEQ ID NO: 13** shows the DNA sequence of the anti-CEA scFv that was cloned into the PEantiC constructPEantiCP28S.
**SEQ ID NO: 14** shows the DNA sequence of the linker between the anti-CEA scFv and fragment of Exotoxin A (Domains II, IB and III) as encoded by the PEantiC construct PEantiCP28S.
**SEQ ID NO: 15** shows the DNA sequence of the fragment of Exotoxin A (Domains II, IB and III) that was cloned into the PEantiC construct PEantiCP28S.
**SEQ ID NO: 16** shows the DNA sequence of the PEantiC immunotoxin: the anti-CEA scFv linked via a linker to a fragment of Exotoxin A (Domains II, IB and III), that was cloned into the PEantiC construct.

## Claims

1. A method for purifying a protein comprising an Exotoxin A, Exotoxin A mutein or active fragment thereof, the method comprising:
(a) solubilizing a sample comprising the protein in a solubilization buffer containing urea and at alkaline pH, the protein is a recombinant protein, sample is a bacterial pellet obtained following expression of the recombinant protein in bacteria, wherein the solubilization buffer contains urea at a concentration in the range of from 1.8 to 2.2M and has a buffer pH in the range of from 11.5 to 12.5, and wherein the solubilization buffer is a phosphate buffer,
(b) decreasing the concentration of urea and the pH by dilution using a dilution buffer, prior to purification of the protein, and wherein the dilution buffer comprises phosphate buffer without urea at a pH in the range of from pH 7.5 to 8.5, preferably wherein three volumes of dilution buffer are added to one volume of lysate.
(c) purifying a protein by chromatographic separation, wherein purification is performed by chromatographic separation by passing the diluted protein through one immobilized metal ion affinity chromatography (IMAC) column or one IMAC and one chitin column, and wherein the elution buffer for IMAC comprises 40 to 60 mM NaH₂PO₄, 270 to 330mM NaCl, 450 to 550mM imidazole, 9 to 11% glycerol at pH 7.5 to 8.5.

2. The method according to claim 1, wherein the solubilization buffer comprises 2M urea and the buffer pH is about 12.0.

3. The method according to claims 1-2, wherein the bacteria is an *E. coli* strain, even more preferably the bacteria is *E. coli* strain NiCo21(DE3) or NiCo23(DE3).

4. The method according to any one of the preceding claims, wherein the solubilization buffer comprises 40 to 60mM NaH₂PO₄, 270 to 330mM NaCl, 18 to 22mM imidazole, 9 to 11% glycerol, 1.8 to 2.2M urea at a pH in the range of from pH11.5 to 12.5;
preferably wherein the solubilization buffer comprises 50mM NaH₂PO₄, 300mM NaCl, 20mM imidazole, 10% glycerol, 0.5mM PMSF, 5mM β-mercaptoethanol, 2M urea at pH12.0;
preferably wherein the solubilization buffer comprises 50mM NaH₂PO₄, 300mM NaCl, 20mM imidazole, 10% glycerol, 0.5mM PMSF, 5mM β-mercaptoethanol, lysozyme (1mg/ml), 0.05% Triton™ X-100, benzonase (5U/ml), 2M urea at pH12.0.

5. The method according to any one of the preceding claims, wherein the dilution buffer comprises 40 to 60 mM NaH₂PO₄, 270 to 330mM NaCl, 18 to 22mM imidazole, 9 to 11 % glycerol at a pH in the range of from pH 7.5 to 8.5;
preferably wherein the dilution buffer comprises 50mM NaH₂PO₄, 300mM NaCl, 20mM imidazole, 10% glycerol at pH 8.0.

6. The method according to any one of claims 1 to 4, wherein purification is performed by chromatographic separation by passing the protein through two columns wherein the protein is first passed through a chitin column and secondly through a nickel, cobalt or copper column,
preferably wherein purification is performed by chromatographic separation by passing the protein through two connected chromatographic columns; preferably wherein the two connected columns are a chitin column and an IMAC nickel, cobalt or copper column.

7. The method according to any one of claims 1 to 6, wherein the elution buffer for IMAC comprises 50mM NaH₂PO₄, 300mM NaCl, 500mM imidazole, 10% glycerol at pH 8.0.

8. The method according to any one of the preceding claims wherein the protein is a Exotoxin A fusion protein;
preferably protein is a fusion protein comprising a targeting moiety, cell surface receptor ligand or signaling molecule;
preferably wherein the protein is a fusion protein comprising an antibody or antigen-binding fragment thereof, wherein the protein is provided as a His-tagged SUMO protein;
preferably wherein the protein is provided as a His-tagged SUMO protein and after purification by IMAC the His-tagged SUMO protein moiety is removed by incubation with a specific SUMO (ULP-1) protease.

9. The method according to any one of the preceding claims, further comprising separating the protein by size-exchange chromatography (SEC);
preferably wherein SEC is combined with buffer exchange;
preferably wherein the buffer for buffer exchange is into PBS, optionally comprising glycerol;
preferably wherein the buffer for buffer exchange is into PBS comprising 10% glycerol.

10. The method according to any one of the preceding claims further comprising concentrating the protein obtained by lyophilisation, spray drying, ultrafiltration or diafiltration.

11. The method according to any one of the preceding claims wherein the Exotoxin A, Exotoxin A mutein or fragment thereof is an active Exotoxin A, active Exotoxin A mutein, active fragment of Exotoxin A or active fragment of an Exotoxin A mutein.

12. The method according to any one of the preceding claims further comprising testing the activity of the protein obtained;
preferably comprising testing the cytotoxic activity of the protein obtained;
preferably comprising testing the cytotoxic activity of the protein obtained by ADP-ribosylation assay and / or by neutral red uptake assay.

13. The method according to any one of claims 1 to 12, further comprising conjugating the protein obtained with a targeting moiety, cell surface receptor ligand, signaling molecule or further cytotoxin.

14. The method according to any one of the preceding claims further comprising formulating the protein or conjugate obtained into a composition with one or more excipients;
preferably further comprising formulating the protein or conjugate obtained into a pharmaceutical composition with one or more pharmaceutically-acceptable excipients.

## Patentansprüche

1. Verfahren zur Reinigung eines Proteins, enthaltend ein Exotoxin A, Exotoxin A Mutein oder ein aktives Fragment davon, wobei das Verfahren umfasst:
(a) Solubilisieren einer Probe, die das Protein enthält, in einem Solubilisierungspuffer, der Harnstoff enthält, und bei alkalischem pH-Wert, wobei das Protein ein rekombinantes Protein ist, die Probe ein bakterielles Pellet ist, das nach der Expression des rekombinanten Proteins in Bakterien erhalten wird, wobei der Solubilisierungspuffer Harnstoff in einer Konzentration im Bereich von 1,8 bis 2,2 M enthält und einen Puffer-pH-Wert im Bereich von 11,5 bis 12,5 aufweist, und wobei der Solubilisierungspuffer ein Phosphatpuffer ist,
(b) Verminderung der Harnstoffkonzentration und des pH-Wertes durch Verdünnung unter Verwendung eines Verdünnungspuffers vor der Reinigung des Proteins, und wobei der Verdünnungspuffer Phosphatpuffer ohne Harnstoff bei pH im Bereich von pH 7,5 bis 8,5 enthält, wobei vorzugsweise drei Volumina der Verdünnungspuffers zu einem Volumen des Lysats zugegeben werden.
(c) Reinigen eines Proteins durch chromatographische Trennung, wobei die Reinigung durch chromatographische Trennung durchgeführt wird, indem das verdünnte Protein durch eine Säule für Immobilisierte-Metallionen-Affinitätschromatographie (IMAC) oder eine IMAC und eine Chitinsäule geleitet wird, und wobei der Elutionspuffer für IMAC 40 bis 60 mM NaH₂PO₄, 270 bis 330 mM NaCl, 450 bis 550 mM Imidazol, 9 bis 11% Glycerin bei pH 7,5 bis 8,5 enthält.

2. Verfahren nach Anspruch 1, wobei der Solubilisierungspuffer 2 M Harnstoff enthält und pH des Puffers etwa 12,0 beträgt.

3. Verfahren nach Anspruch 1-2, wobei das Bakterium ein *E. coli*-Stamm ist, noch bevorzugter das Bakterium der *E. coli*-Stamm NiCo21(DE3) oder NiCo23(DE3) ist.

4. Verfahren nach einem der vorstehenden Ansprüche, wobei der Solubilisierungspuffer 40 bis 60 mM NaH₂PO₄, 270 bis 330 mM NaCl, 18 bis 22 mM Imidazol, 9 bis 11% Glycerin, 1,8 bis 2,2 M Harnstoff bei pH im Bereich von pH 11,5 bis 12,5 enthält;
wobei der Solubilisierungspuffer bevorzugt 50 mM NaH₂PO₄, 300 mM NaCl, 20 mM Imidazol, 10% Glycerin, 0,5 mM PMSF, 5 mM β-Mercaptoethanol, 2M Harnstoff bei pH 12,0 enthält;
wobei der Solubilisierungspuffer bevorzugt 50 mM NaH₂PO₄, 300 mM NaCl, 20 mM Imidazol, 10% Glycerin, 0,5 mM PMSF, 5 mM β-Mercaptoethanol, Lysozym (1 mg/ml), 0,05% Triton™ X-100, Benzonase (5 U/ml), 2M Harnstoff bei pH 12,0 enthält.

5. Verfahren nach einem der vorstehenden Ansprüche, wobei der Verdünnungspuffer 40 bis 60 mM NaH₂PO₄, 270 bis 330 mM NaCl, 18 bis 22 mM Imidazol, 9 bis 11% Glycerin bei pH im Bereich von pH 7,5 bis 8,5 enthält;
wobei der Verdünnungspuffer bevorzugt 50 mM NaH₂PO₄, 300 mM NaCl, 20 mM Imidazol, 10% Glycerin bei pH 8,0 enthält.

6. Verfahren nach einem der Ansprüche 1 bis 4, wobei die Reinigung durch chromatographische Trennung durchgeführt wird, indem das Protein durch zwei Säulen geleitet wird, wobei das Protein zuerst durch eine Chitinsäule und zweitens durch eine Nickel-, Kobalt- oder Kupfersäule geleitet wird,
wobei die Reinigung bevorzugt durch chromatographische Trennung durchgeführt wird, indem das Protein durch zwei verbundene chromatographische Säulen geleitet wird; wobei die zwei verbundenen Säulen vorzugsweise eine Chitinsäule und eine IMAC-Nickel-, Kobalt- oder Kupfersäule sind.

7. Verfahren nach einem der Ansprüche 1 bis 6, wobei der Elutionspuffer für IMAC 50 mM NaH₂PO₄, 300 mM NaCl, 500 mM Imidazol, 10% Glycerin bei pH 8,0 enthält.

8. Verfahren nach einem der vorstehenden Ansprüche, wobei das Protein ein Exotoxin A-Fusionsprotein ist;
bevorzugt ein Protein ein Fusionsprotein ist, das eine Targeting-Einheit, einen Zelloberflächenrezeptorliganden oder ein Signalmolekül enthält;
wobei bevorzugt das Protein ein Fusionsprotein umfassend einen Antikörper oder ein Antigen-bindendes Fragment davon ist, wobei das Protein als ein His-markiertes SUMO-Protein bereitgestellt wird;
wobei bevorzugt das Protein als ein His-markiertes SUMO-Protein bereitgestellt wird und nach Reinigung durch IMAC der His-markiertes SUMO-Proteineinheit durch Inkubation mit einer spezifischen SUMO-Protease (ULP-1) entfernt wird.

9. Verfahren nach einem der vorstehenden Ansprüche, das ferner die Trennung des Proteins durch Größenausschlusschromatographie (SEC) umfasst;
wobei SEC bevorzugt mit Pufferaustausch kombiniert wird;
wobei der Puffer für den Pufferaustausch bevorzugt in PBS vorliegt, das gegebenenfalls Glycerin enthält;
wobei der Puffer für den Pufferaustausch bevorzugt in PBS mit 10% Glycerin vorliegt.

10. Verfahren nach einem der vorstehenden Ansprüche ferner die Konzentrierung des durch Lyophilisation, Sprühtrocknung, Ultrafiltration oder Diafiltration gewonnenen Proteins umfasst.

11. Verfahren nach einem der vorstehenden Ansprüche, wobei das Exotoxin A, Exotoxin A-Mutein oder Fragment davon ist, ein aktives Exotoxin A, aktives Exotoxin A-Mutein, aktives Fragment von Exotoxin A oder aktives Fragment eines Exotoxin A-Muteins ist.

12. Verfahren nach einem der vorstehenden Ansprüche umfassend ferner die Prüfung der Proteinaktivität durch Größenausschlusschromatographie (SEC);
umfassend bevorzugt die Prüfung der zytotoxischen Aktivität des erhaltenen Proteins;
umfassend bevorzugt die Prüfung der zytotoxischen Aktivität des Proteins, das durch einen ADP-Ribosylierung-Test und/oder durch einen Neutralrot-Test erhalten wurde.

13. Verfahren nach einem der Ansprüche 1 bis 12, umfassend ferner das Konjugieren des erhaltenen Proteins mit einer Targeting-Einheit, einem Zelloberflächenrezeptorliganden, einem Signalmolekül oder einem weiteren Zytotoxin.

14. Verfahren nach einem der vorstehenden Ansprüche umfassend ferner das Formulieren des erhaltenen Proteins oder Konjugats zu einer Zusammensetzung mit einem oder mehreren Hilfsstoffen;
bevorzugt umfassend ferner das Formulieren des erhaltenen Proteins oder Konjugats zu einer pharmazeutischen Zusammensetzung mit einem oder mehreren pharmazeutisch annehmbaren Hilfsstoffen.

## Revendications

1. Une méthode de purification d'une protéine comprenant une exotoxine A, une mutéine d'exotoxine A ou un fragment actif de celle-ci, la méthode comprenant :
(a) solubilisation d'un échantillon comprenant la protéine dans un tampon de solubilisation contenant de l'urée et à un pH alcalin, la protéine est une protéine recombinante, l'échantillon est un culot bactérien obtenu suivant l'expression de la protéine recombinante dans des bactéries, dans laquelle le tampon de solubilisation contient de l'urée à une concentration dans la gamme de 1,8 à 2,2 M et a un pH de tampon dans la gamme de 11,5 à 12,5, et dans laquelle le tampon de solubilisation est un tampon au phosphate,
(b) diminution de la concentration de l'urée et du pH par dilution en utilisant un tampon de dilution, avant la purification de la protéine, et dans laquelle le tampon de dilution comprend le tampon au phosphate sans urée à un pH dans la gamme de pH 7,5 à 8,5, de préférence dans laquelle trois volumes du tampon de dilution sont ajoutés à un volume de lysat.
(c) purification d'une protéine par séparation chromatographique, dans laquelle la purification est effectuée par séparation chromatographique en faisant passer la protéine diluée à travers une colonne de chromatographie d'affinité aux ions métalliques immobilisés (IMAC) ou une colonne d'IMAC et une colonne de chitine, et dans laquelle le tampon d'élution pour IMAC comprend 40 à 60 mM de NaH₂PO₄, 270 à 330 mM de NaCl, 450 à 550 mM d'imidazole, 9 à 11% de glycérol à pH 7,5 à 8,5.

2. La méthode selon la revendication 1, dans laquelle le tampon de solubilisation comprend l'urée 2M et le pH du tampon est d'environ 12,0.

3. La méthode selon les revendications 1-2, dans laquelle la bactérie est une souche d'*E. Coli*, encore plus préférablement la bactérie est la souche d'*E. Coli* NiCo21(DE3) ou NiCo23(DE3).

4. La méthode selon l'une quelconque des revendications précédentes, dans laquelle le tampon de solubilisation comprend 40 à 60 mM de NaH₂PO₄, 270 à 330 mM de NaCl, 18 à 22 mM d'imidazole, 9 à 11% de glycérol, 1,8 à 2,2 M d'urée à un pH dans la plage de pH 11,5 à 12,5 ;
de préférence dans laquelle le tampon de solubilisation comprend 50 mM de NaH₂PO₄, 300 mM de NaCl, 20 mM d'imidazole, 10% de glycérol, 0,5 mM de PMSF, 5 mM de β-mercaptoéthanol, 2 M d'urée à pH 12,0 ;
de préférence dans laquelle le tampon de solubilisation comprend 50 mM de NaH₂PO₄, 300 mM de NaCl, 20 mM d'imidazole, 10% de glycérol, 0,5 mM de PMSF, 5 mM de β-mercaptoéthanol, lysozyme (1mg/ml), Triton™ X-100 0,05%, benzonase (5 U/ml), urée 2M à pH 12,0.

5. La méthode selon l'une quelconque des revendications précédentes, dans laquelle le tampon de dilution comprend 40 à 60 mM de NaH₂PO₄, 270 à 330 mM de NaCl, 18 à 22 mM d'imidazole, 9 à 11% de glycérol à un pH dans la plage de pH 7,5 à 8,5 ;
de préférence dans laquelle le tampon de dilution comprend 50 mM de NaH₂PO₄, 300 mM de NaCl, 20 mM d'imidazole, 10% de glycérol à pH 8,0.

6. La méthode selon l'une quelconque des revendications 1 à 4, dans laquelle la purification est effectuée par séparation chromatographique en faisant passer la protéine à travers deux colonnes, dans laquelle la protéine est d'abord passée à travers une colonne de chitine et ensuite à travers une colonne de nickel, de cobalt ou de cuivre,
de préférence dans laquelle la purification est effectuée par séparation chromatographique en faisant passer la protéine à travers deux colonnes chromatographiques connectées; de préférence dans laquelle les deux colonnes connectées sont une colonne de chitine et une colonne IMAC de nickel, de cobalt ou de cuivre.

7. La méthode selon l'une quelconque des revendications 1 à 6, dans laquelle le tampon d'élution pour IMAC comprend 50 mM de NaH₂PO₄, 300 mM de NaCl, 500 mM d'imidazole, 10% de glycérol à pH 8,0.

8. La méthode selon l'une quelconque des revendications précédentes, dans laquelle la protéine est une protéine de fusion d'exotoxine A ;
de préférence, la protéine est une protéine de fusion comprenant un groupement de ciblage, un ligand de récepteur de surface cellulaire ou une molécule de signalisation ;
de préférence dans laquelle la protéine est une protéine de fusion comprenant un anticorps ou un fragment de liaison à l'antigène de celui-ci, dans laquelle la protéine est fournie sous forme d'une protéine SUMO marquée His ;
de préférence dans laquelle la protéine est fournie sous forme d'une protéine SUMO marquée His et après purification par IMAC, le groupement protéique SUMO marqué His est éliminé par incubation avec une protéase SUMO (ULP-1) spécifique.

9. La méthode selon l'une quelconque des revendications précédentes, comprenant en outre la séparation de la protéine par chromatographie d'échange de taille (SEC) ;
de préférence dans laquelle SEC est combinée avec un échange de tampon ;
de préférence dans laquelle le tampon pour l'échange de tampon est en PBS, comprenant facultativement du glycérol ;
de préférence dans laquelle le tampon pour l'échange de tampon est en PBS comprenant 10% de glycérol.

10. La méthode selon l'une quelconque des revendications précédentes, comprenant en outre la concentration de la protéine obtenue par lyophilisation, séchage par pulvérisation, ultrafiltration ou diafiltration.

11. La méthode selon l'une quelconque des revendications précédentes, dans laquelle l'exotoxine A, la mutéine d'exotoxine A ou un fragment de celle-ci est une exotoxine A active, une mutéine d'exotoxine A active, un fragment actif d'exotoxine A ou un fragment actif d'une mutéine d'exotoxine A.

12. La méthode selon l'une quelconque des revendications précédentes, comprenant en outre le test de l'activité de la protéine obtenue ;
comprenant de préférence le test de l'activité cytotoxique de la protéine obtenue ;
comprenant de préférence le test de l'activité cytotoxique de la protéine obtenue par essai de ribosylation ADP et/ou par essai d'absorption rouge neutre.

13. La méthode selon l'une quelconque des revendications 1 à 12, comprenant en outre la conjugaison de la protéine obtenue avec un groupement de ciblage, un ligand de récepteur de surface cellulaire, une molécule de signalisation ou une cytotoxine supplémentaire.

14. La méthode selon l'une quelconque des revendications précédentes, comprenant en outre la formulation de la protéine ou du conjugué obtenu dans une composition avec un ou plusieurs excipients ;
de préférence comprenant en outre la formulation de la protéine ou du conjugué obtenu en une composition pharmaceutique avec un ou plusieurs excipients pharmaceutiquement acceptables.
